# EUROPEAN PATENT SPECIFICATION

(11) **EP 3 128 326 B1**
(45) Date of publication and mention of the grant of the patent: **14.03.2018**
(21) Application number: 15772611.8
(22) Date of filing: 03.04.2015
(51) Int. Cl.: G01N 33/68, C07K 14/705, C12N 1/15, C12N 1/19, C12N 1/21, C12N 5/10, C12N 9/12, C12N 15/09, C12Q 1/68, G01N 33/15, G01N 33/50

(54) **BIOMARKER FOR DIAGNOSIS OF AGING OR AMYOTROPHIA**
BIOMARKER ZUR DIAGNOSE DES ALTERNS ODER VON AMYOTROPHIA
BIOMARQUEUR POUR DIAGNOSTIC DE VIEILLISSEMENT OU D'AMYOTROPHIE

(30) Priority: 03.04.2014 JP 2014077086
(43) Date of publication of application: 08.02.2017
(73) Proprietor: Tokyo Metropolitan Geriatric Hospital and Institute of Gerontology, Tokyo 173-0015 (JP)
(72) Inventor: SHIGEMOTO, Kazuhiro, Tokyo 173-0015 (JP)
(74) Representative: Cohausz & Florack
(86) International application number: PCT/JP2015/060671
(87) International publication number: WO 2015/152413

(56) References cited:
- WO-A1-2007/141971
- WO-A1-2009/101968
- WO-A1-2013/108926
- WO-A2-2011/036297
- JP-A- 2000 504 929
- JP-A- 2009 189 268
- JP-A- 2011 173 793
- JP-A- 2013 100 275
- JP-A- 2013 503 825
- US-A1- 2002 164 702
- US-A1- 2004 082 010
- KUEHN ET AL: "Multiple alternatively spliced transcripts of the receptor tyrosine kinase MuSK are expressed in muscle", GENE, ELSEVIER, AMSTERDAM, NL, vol. 360, no. 2, 7 November 2005 (2005-11-07), pages 83-91, XP005116360, ISSN: 0378-1119, DOI: 10.1016/J.GENE.2005.07.009
- KAZUHIRO SHIGEMOTO: 'Koreisha ni Okeru Kareisei Kinniku Genjaku Gensho (Sarcopenia) ni Kansuru Yobo Taisaku Kakuritsu no Tameno Hokatsuteki Kenkyu Sarcopenia no Biomarker no Kaihatsu to Jitsuyoka' KOREISHA NI OKERU KAREISEI KINNIKU GENJAKU GENSHO (SARCOPENIA) NI KANSURU YOBO TAISAKU KAKURITSU NO TAMENO HOKATSUTEKI KENKYU HEISEI 23 NENDO SOKATSU BUNTAN KENKYU HOKOKUSHO 2012, pages 63 - 69, XP008182675
- VALENZUELA D M ET AL.: 'Receptor tyrosine kinase specific for the skeletal muscle lineage: expression in embryonic muscle, at the neuromuscular junction, and after injury' NEURON vol. 15, no. 3, 1995, pages 573 - 584, XP000674260
- MASAKATSU MOTOMURA ET AL.: 'Men'ekisei Shinkei Shikkan ni Kansuru Chosa Kenkyu Jiko Men'ekisei MuSK Kotai Yosei Jusho Kin Muryokusho Rat Sakusei no Kokoromi' MEN'EKISEI SHINKEI SHIKKAN NI KANSURU CHOSA KENKYU HEISEI 17 NENDO SOKATSU BUNTAN KENKYU HOKOKUSHO 2006, pages 131 - 132, XP008182676
- HIROFUMI MIYATA: 'Rat Endplate ni Okeru Synapse Kaku Domain to MuSK Hatsugen no Karei Henka' JAPANESE JOURNAL OF PHYSICAL FITNESS AND SPORTS MEDICINE vol. 60, no. 6, 2011, page 605, XP008182506
- KAZUHIRO SHIGEMOTO: 'Koreisha ni Okeru Kareisei Kinniku Genjaku Gensho (Sarcopenia) ni Kansuru Yobo Taisaku Kakuritsu no Tameno Hokatsuteki Kenkyu Sarcopenia no Biomarker no Kaihatsu to Jitsuyoka' KOREISHA NI OKERU KAREISEI KINNIKU GENJAKU GENSHO (SARCOPENIA) NI KANSURU YOBO TAISAKU KAKURITSU NO TAMENO HOKATSUTEKI KENKYU HEISEI 24 NENDO SOKATSU BUNTAN KENKYU HOKOKUSHO 2013, pages 75 - 81, XP008182674
- XIE M-H ET AL.: 'Direct demonstration of MuSK involvement in acetylcholine receptor clustering through identification of agonist ScFv' NAT BIOTECHNOL vol. 15, no. 8, 1997, ISSN 1087-0156 pages 768 - 771, XP002095742
- TSUYOSHI ENDO: 'Elucidation of signal transduction mechanism in cell fusion (Foundation for the Promotion Res. on Medical Resources S' RESEARCH REPORT SUPPORTED BY GRANTS FROM THE FOUNDATION FOR PROMOTION RES. ON MEDICAL RESOURCES vol. 1997, 1996, pages 13 - 15, XP008183369
- SHIGEMOTO KAZUHIRO ET AL.: 'Induction of myasthenia by immunization against muscle- specific kinase' THE JOURNAL OF CLINICAL INVESTIGATION vol. 116, no. 4, 2006, ISSN 0021-9738 pages 1016 - 1024, XP055229841

### SEQUENCE LISTING

<110> Tokyo Metropolitan Geriatric Hospital and Institute of Gerontology
<120> Biomarker for diagnosis of aging or amyotrophy
<130> TMI-961
<150> JP 2014-077086
   <151> 2014-04-03
<160> 12
<170> PatentIn version 3.5
<210> 1
   <211> 1395
   <212> DNA
   <213> homo sapiens
<220>
   <221> CDS
   <222> (1)..(1395)
<400> 1
   atg aga gag ctc gtc aac att cca ctg gta cat att ctt act ctg gtt      48
   Met Arg Glu Leu Val Asn Ile Pro Leu Val His Ile Leu Thr Leu Val
   1               5                   10                  15
   gcc ttc agc gga act gag aaa ctt cca aaa gct cct gtc atc acc act      96
   Ala Phe Ser Gly Thr Glu Lys Leu Pro Lys Ala Pro Val Ile Thr Thr
               20                  25                  30
   cct ctt gaa aca gtg gat gcc tta gtt gaa gaa gtg gct act ttc atg      144
   Pro Leu Glu Thr Val Asp Ala Leu Val Glu Glu Val Ala Thr Phe Met
           35                  40                  45
   tgt gca gtg gaa tcc tac ccc cag cct gag att tcc tgg act aga aat      192
   Cys Ala Val Glu Ser Tyr Pro Gln Pro Glu Ile Ser Trp Thr Arg Asn
       50                  55                  60
   aaa att ctc att aaa ctc ttt gac acc cgg tac agc atc cgg gag aat      240
   Lys Ile Leu Ile Lys Leu Phe Asp Thr Arg Tyr Ser Ile Arg Glu Asn
   65                  70                  75                  80
   ggg cag ctc ctc acc atc ctg agt gtg gaa gac agt gat gat ggc att      288
   Gly Gln Leu Leu Thr Ile Leu Ser Val Glu Asp Ser Asp Asp Gly Ile
                   85                  90                  95
   tac tgc tgc acg gcc aac aat ggt gtg gga gga gct gtg gag agt tgt      336
   Tyr Cys Cys Thr Ala Asn Asn Gly Val Gly Gly Ala Val Glu Ser Cys
               100                 105                 110
   gga gcc ctg caa gtg aag atg aaa cct aaa ata act cgc cct ccc ata      384
   Gly Ala Leu Gln Val Lys Met Lys Pro Lys Ile Thr Arg Pro Pro Ile
           115                 120                 125
   aat gtg aaa ata ata gag gga tta aaa gca gtc cta cca tgt act aca      432
   Asn Val Lys Ile Ile Glu Gly Leu Lys Ala Val Leu Pro Cys Thr Thr
       130                 135                 140
   atg ggt aat ccc aaa cca tca gtg tct tgg ata aag gga gac agc cct       480
   Met Gly Asn Pro Lys Pro Ser Val Ser Trp Ile Lys Gly Asp Ser Pro
   145                 150                 155                 160
   ctc agg gaa aat tcc cga att gca gtt ctt gaa tct ggg agc ttg agg       528
   Leu Arg Glu Asn Ser Arg Ile Ala Val Leu Glu Ser Gly Ser Leu Arg
                   165                 170                 175
   att cat aac gta caa aag gaa gat gca gga cag tat cga tgt gtg gca       576
   Ile His Asn Val Gln Lys Glu Asp Ala Gly Gln Tyr Arg Cys Val Ala
               180                 185                 190
   aaa aac agc ctc ggg aca gca tat tcc aaa gtg gtg aag ctg gaa gtt       624
   Lys Asn Ser Leu Gly Thr Ala Tyr Ser Lys Val Val Lys Leu Glu Val
           195                 200                 205
   gag gtt ttt gcc agg atc ctg cgg gct cct gaa tcc cac aat gtc acc       672
   Glu Val Phe Ala Arg Ile Leu Arg Ala Pro Glu Ser His Asn Val Thr
       210                 215                 220
   ttt ggc tcc ttt gtg acc ctg cac tgt aca gca aca ggc att cct gtc       720
   Phe Gly Ser Phe Val Thr Leu His Cys Thr Ala Thr Gly Ile Pro Val
   225                 230                 235                 240
   ccc acc atc acc tgg att gaa aac gga aat gct gtt tct tct ggg tcc       768
   Pro Thr Ile Thr Trp Ile Glu Asn Gly Asn Ala Val Ser Ser Gly Ser
                   245                 250                 255
   att caa gag agt gtg aaa gac cga gtg att gac tca aga ctg cag ctg       816
   Ile Gln Glu Ser Val Lys Asp Arg Val Ile Asp Ser Arg Leu Gln Leu
               260                 265                 270
   ttt atc acc aag cca gga ctc tac aca tgc ata gct acc aat aag cat       864
   Phe Ile Thr Lys Pro Gly Leu Tyr Thr Cys Ile Ala Thr Asn Lys His
           275                 280                 285
   ggg gag aag ttc agt act gcc aag gct gca gcc acc atc agc ata gca       912
   Gly Glu Lys Phe Ser Thr Ala Lys Ala Ala Ala Thr Ile Ser Ile Ala
       290                 295                 300
   gaa tgg agt aaa cca cag aaa gat aac aaa ggc tac tgc gcc cag tac       960
   Glu Trp Ser Lys Pro Gln Lys Asp Asn Lys Gly Tyr Cys Ala Gln Tyr
   305                 310                 315                 320
   aga ggg gag gtg tgt aat gca gtc ctg gca aaa gat gct ctt gtt ttt      1008
   Arg Gly Glu Val Cys Asn Ala Val Leu Ala Lys Asp Ala Leu Val Phe
                   325                 330                 335
   ctc aac acc tcc tat gcg gac cct gag gag gcc caa gag cta ctg gtc      1056
   Leu Asn Thr Ser Tyr Ala Asp Pro Glu Glu Ala Gln Glu Leu Leu Val
               340                 345                 350
   cac acg gcc tgg aat gaa ctg aaa gta gtg agc cca gtc tgc cgg cca      1104
   His Thr Ala Trp Asn Glu Leu Lys Val Val Ser Pro Val Cys Arg Pro
           355                 360                 365
   gct gct gag gct ttg ttg tgt aac cac atc ttc cag gag tgc agt cct      1152
   Ala Ala Glu Ala Leu Leu Cys Asn His Ile Phe Gln Glu Cys Ser Pro
       370                 375                 380
   gga gta gtg cct act cct att ccc att tgc aga gag tac tgc ttg gca      1200
   Gly Val Val Pro Thr Pro Ile Pro Ile Cys Arg Glu Tyr Cys Leu Ala
   385                 390                 395                 400
   gta aag gag ctc ttc tgc gca aaa gaa tgg ctg gta atg gaa gag aag      1248
   Val Lys Glu Leu Phe Cys Ala Lys Glu Trp Leu Val Met Glu Glu Lys
                   405                 410                 415
   acc cac aga gga ctc tac aga tcc gag atg cat ttg ctg tcc gtg cca      1296
   Thr His Arg Gly Leu Tyr Arg Ser Glu Met His Leu Leu Ser Val Pro
               420                 425                 430
   gaa tgc agc aag ctt ccc agc atg cat tgg gac ccc acg gcc tgt gcc      1344
   Glu Cys Ser Lys Leu Pro Ser Met His Trp Asp Pro Thr Ala Cys Ala
           435                 440                 445
   aga ctg cca cat cta ggt aac aca gag ttc tcc caa gac ttt gga ggt      1392
   Arg Leu Pro His Leu Gly Asn Thr Glu Phe Ser Gln Asp Phe Gly Gly
       450                 455                 460
   taa                                                                  1395
<210> 2
   <211> 464
   <212> PRT
   <213> homo sapiens
<400> 2
   Met Arg Glu Leu Val Asn Ile Pro Leu Val His Ile Leu Thr Leu Val
   1               5                   10                  15
   Ala Phe Ser Gly Thr Glu Lys Leu Pro Lys Ala Pro Val Ile Thr Thr
               20                  25                  30
   Pro Leu Glu Thr Val Asp Ala Leu Val Glu Glu Val Ala Thr Phe Met
           35                  40                  45
   Cys Ala Val Glu Ser Tyr Pro Gln Pro Glu Ile Ser Trp Thr Arg Asn
       50                  55                  60
   Lys Ile Leu Ile Lys Leu Phe Asp Thr Arg Tyr Ser Ile Arg Glu Asn
   65                  70                  75                  80
   Gly Gln Leu Leu Thr Ile Leu Ser Val Glu Asp Ser Asp Asp Gly Ile
                   85                  90                  95
   Tyr Cys Cys Thr Ala Asn Asn Gly Val Gly Gly Ala Val Glu Ser Cys
               100                 105                 110
   Gly Ala Leu Gln Val Lys Met Lys Pro Lys Ile Thr Arg Pro Pro Ile
           115                 120                 125
   Asn Val Lys Ile Ile Glu Gly Leu Lys Ala Val Leu Pro Cys Thr Thr
       130                 135                 140
   Met Gly Asn Pro Lys Pro Ser Val Ser Trp Ile Lys Gly Asp Ser Pro
   145                 150                 155                 160
   Leu Arg Glu Asn Ser Arg Ile Ala Val Leu Glu Ser Gly Ser Leu Arg
                   165                 170                 175
   Ile His Asn Val Gln Lys Glu Asp Ala Gly Gln Tyr Arg Cys Val Ala
               180                 185                 190
   Lys Asn Ser Leu Gly Thr Ala Tyr Ser Lys Val Val Lys Leu Glu Val
           195                 200                 205
   Glu Val Phe Ala Arg Ile Leu Arg Ala Pro Glu Ser His Asn Val Thr
       210                 215                 220
   Phe Gly Ser Phe Val Thr Leu His Cys Thr Ala Thr Gly Ile Pro Val
   225                 230                 235                 240
   Pro Thr Ile Thr Trp Ile Glu Asn Gly Asn Ala Val Ser Ser Gly Ser
                   245                 250                 255
   Ile Gln Glu Ser Val Lys Asp Arg Val Ile Asp Ser Arg Leu Gln Leu
               260                 265                 270
   Phe Ile Thr Lys Pro Gly Leu Tyr Thr Cys Ile Ala Thr Asn Lys His
           275                 280                 285
   Gly Glu Lys Phe Ser Thr Ala Lys Ala Ala Ala Thr Ile Ser Ile Ala
       290                 295                 300
   Glu Trp Ser Lys Pro Gln Lys Asp Asn Lys Gly Tyr Cys Ala Gln Tyr
   305                 310                 315                 320
   Arg Gly Glu Val Cys Asn Ala Val Leu Ala Lys Asp Ala Leu Val Phe
                   325                 330                 335
   Leu Asn Thr Ser Tyr Ala Asp Pro Glu Glu Ala Gln Glu Leu Leu Val
               340                 345                 350
   His Thr Ala Trp Asn Glu Leu Lys Val Val Ser Pro Val Cys Arg Pro
           355                 360                 365
   Ala Ala Glu Ala Leu Leu Cys Asn His Ile Phe Gln Glu Cys Ser Pro
       370                 375                 380
   Gly Val Val Pro Thr Pro Ile Pro Ile Cys Arg Glu Tyr Cys Leu Ala
   385                 390                 395                 400
   Val Lys Glu Leu Phe Cys Ala Lys Glu Trp Leu Val Met Glu Glu Lys
                   405                 410                 415
   Thr His Arg Gly Leu Tyr Arg Ser Glu Met His Leu Leu Ser Val Pro
               420                 425                 430
   Glu Cys Ser Lys Leu Pro Ser Met His Trp Asp Pro Thr Ala Cys Ala
           435                 440                 445
   Arg Leu Pro His Leu Gly Asn Thr Glu Phe Ser Gln Asp Phe Gly Gly
       450                 455                 460
<210> 3
   <211> 1395
   <212> DNA
   <213> Mus musculus
<220>
   <221> CDS
   <222> (1)..(1395)
<400> 3
   atg aga gag ctt gtc aac att cca ctg tta cag atg ctc acc ctg gtt      48
   Met Arg Glu Leu Val Asn Ile Pro Leu Leu Gln Met Leu Thr Leu Val
   1               5                   10                  15
   gcc ttc agc ggg act gag aaa ctt cca aaa gcc cct gtc atc acc acg      96
   Ala Phe Ser Gly Thr Glu Lys Leu Pro Lys Ala Pro Val Ile Thr Thr
               20                  25                  30
   cct ctt gaa act gta gat gcc ttg gtt gaa gaa gta gcg act ttc atg      144
   Pro Leu Glu Thr Val Asp Ala Leu Val Glu Glu Val Ala Thr Phe Met
           35                  40                  45
   tgt gcc gtg gaa tcc tac cct cag ccc gag att tct tgg acc aga aat      192
   Cys Ala Val Glu Ser Tyr Pro Gln Pro Glu Ile Ser Trp Thr Arg Asn
       50                  55                  60
   aaa att ctc att aag ctg ttt gac acc cgc tac agc atc cgg gag aat      240
   Lys Ile Leu Ile Lys Leu Phe Asp Thr Arg Tyr Ser Ile Arg Glu Asn
   65                  70                  75                  80
   ggt cag ctc ctc acc att ctg agc gtg gaa gac agt gat gat ggc atc      288
   Gly Gln Leu Leu Thr Ile Leu Ser Val Glu Asp Ser Asp Asp Gly Ile
                   85                  90                  95
   tac tgc tgc ata gcc aac aat gga gtg gga gga gcc gtg gag agt tgt      336
   Tyr Cys Cys Ile Ala Asn Asn Gly Val Gly Gly Ala Val Glu Ser Cys
               100                 105                 110
   ggt gcc ctg caa gtg aag atg aaa cct aaa ata act cgt cct ccc att      384
   Gly Ala Leu Gln Val Lys Met Lys Pro Lys Ile Thr Arg Pro Pro Ile
           115                 120                 125
   aat gta aaa ata ata gag gga ttg aag gca gtt ctg ccg tgc act acg      432
   Asn Val Lys Ile Ile Glu Gly Leu Lys Ala Val Leu Pro Cys Thr Thr
       130                 135                 140
   atg ggt aac ccc aaa cca tct gtg tcc tgg atc aag ggg gac aat gct        480
   Met Gly Asn Pro Lys Pro Ser Val Ser Trp Ile Lys Gly Asp Asn Ala
   145                 150                 155                 160
   ctc agg gaa aat tcc aga atc gca gtt ctt gaa tct ggg agc tta agg        528
   Leu Arg Glu Asn Ser Arg Ile Ala Val Leu Glu Ser Gly Ser Leu Arg
                   165                 170                 175
   atc cat aat gtg caa aag gaa gat gca gga cag tac cgc tgt gtg gcc        576
   Ile His Asn Val Gln Lys Glu Asp Ala Gly Gln Tyr Arg Cys Val Ala
               180                 185                 190
   aaa aac agc ctg ggc aca gct tac tcc aaa ctg gtg aag ctg gaa gtg        624
   Lys Asn Ser Leu Gly Thr Ala Tyr Ser Lys Leu Val Lys Leu Glu Val
           195                 200                 205
   gag gtt ttt gca aga atc ctg cgt gct cct gaa tcc cac aat gtc acc        672
   Glu Val Phe Ala Arg Ile Leu Arg Ala Pro Glu Ser His Asn Val Thr
       210                 215                 220
   ttt ggt tcc ttt gta acc cta cgc tgc aca gca ata ggc atc cct gtc        720
   Phe Gly Ser Phe Val Thr Leu Arg Cys Thr Ala Ile Gly Ile Pro Val
   225                 230                 235                 240
   ccc acc atc agc tgg att gaa aac gga aat gct gtt tct tca ggt tcc        768
   Pro Thr Ile Ser Trp Ile Glu Asn Gly Asn Ala Val Ser Ser Gly Ser
                   245                 250                 255
   att caa gag agt gtg aaa gac cga gtg att gac tca aga ctc cag ctc        816
   Ile Gln Glu Ser Val Lys Asp Arg Val Ile Asp Ser Arg Leu Gln Leu
               260                 265                 270
   ttc atc aca aag cca gga ctc tac aca tgc ata gct acc aat aag cac        864
   Phe Ile Thr Lys Pro Gly Leu Tyr Thr Cys Ile Ala Thr Asn Lys His
           275                 280                 285
   gga gaa aag ttc agt acc gca aag gct gca gcc act gtc agc ata gca        912
   Gly Glu Lys Phe Ser Thr Ala Lys Ala Ala Ala Thr Val Ser Ile Ala
       290                 295                 300
   gaa tgg agt aag tca cag aaa gac agc caa ggt tac tgt gcc cag tac        960
   Glu Trp Ser Lys Ser Gln Lys Asp Ser Gln Gly Tyr Cys Ala Gln Tyr
   305                 310                 315                 320
   aga ggg gag gtg tgt gat gca gtc ctg gcg aaa gat gct ctt gtc ttc       1008
   Arg Gly Glu Val Cys Asp Ala Val Leu Ala Lys Asp Ala Leu Val Phe
                   325                 330                 335
   ttc aac acc tcc tac cgg gac ccc gag gac gcc cag gag ctg ctg atc       1056
   Phe Asn Thr Ser Tyr Arg Asp Pro Glu Asp Ala Gln Glu Leu Leu Ile
               340                 345                 350
   cac act gcg tgg aat gag ctg aag gct gtg agt cca ctg tgc cgg cca       1104
   His Thr Ala Trp Asn Glu Leu Lys Ala Val Ser Pro Leu Cys Arg Pro
           355                 360                 365
   gct gct gag gct ctg ctg tgt aac cac ctc ttc caa gag tgc agc cct       1152
   Ala Ala Glu Ala Leu Leu Cys Asn His Leu Phe Gln Glu Cys Ser Pro
       370                 375                 380
   gga gtg gta cct act ccc atg ccc att tgc aga gag tac tgc ctg gcg       1200
   Gly Val Val Pro Thr Pro Met Pro Ile Cys Arg Glu Tyr Cys Leu Ala
   385                 390                 395                 400
   gta aag gag ctc ttc tgt gca aag gaa tgg cag gca atg gaa gga aag      1248
   Val Lys Glu Leu Phe Cys Ala Lys Glu Trp Gln Ala Met Glu Gly Lys
                   405                 410                 415
   gcc cac cgg ggc ctc tac aga tct ggg atg cat ctc ctt ccg gta cca      1296
   Ala His Arg Gly Leu Tyr Arg Ser Gly Met His Leu Leu Pro Val Pro
               420                 425                 430
   gag tgc agc aag ctt ccc agc atg cac cgg gac ccc aca gcc tgc aca      1344
   Glu Cys Ser Lys Leu Pro Ser Met His Arg Asp Pro Thr Ala Cys Thr
           435                 440                 445
   aga ctg cca tat tta ggt aac aaa gaa gtt cct cca gac ttt gga agt      1392
   Arg Leu Pro Tyr Leu Gly Asn Lys Glu Val Pro Pro Asp Phe Gly Ser
       450                 455                 460
   taa                                                                  1395
<210> 4
   <211> 464
   <212> PRT
   <213> Mus musculus
<400> 4
   Met Arg Glu Leu Val Asn Ile Pro Leu Leu Gln Met Leu Thr Leu Val
   1               5                   10                  15
   Ala Phe Ser Gly Thr Glu Lys Leu Pro Lys Ala Pro Val Ile Thr Thr
               20                  25                  30
   Pro Leu Glu Thr Val Asp Ala Leu Val Glu Glu Val Ala Thr Phe Met
           35                  40                  45
   Cys Ala Val Glu Ser Tyr Pro Gln Pro Glu Ile Ser Trp Thr Arg Asn
       50                  55                  60
   Lys Ile Leu Ile Lys Leu Phe Asp Thr Arg Tyr Ser Ile Arg Glu Asn
   65                  70                  75                  80
   Gly Gln Leu Leu Thr Ile Leu Ser Val Glu Asp Ser Asp Asp Gly Ile
                   85                  90                  95
   Tyr Cys Cys Ile Ala Asn Asn Gly Val Gly Gly Ala Val Glu Ser Cys
               100                 105                 110
   Gly Ala Leu Gln Val Lys Met Lys Pro Lys Ile Thr Arg Pro Pro Ile
           115                 120                 125
   Asn Val Lys Ile Ile Glu Gly Leu Lys Ala Val Leu Pro Cys Thr Thr
       130                 135                 140
   Met Gly Asn Pro Lys Pro Ser Val Ser Trp Ile Lys Gly Asp Asn Ala
   145                 150                 155                 160
   Leu Arg Glu Asn Ser Arg Ile Ala Val Leu Glu Ser Gly Ser Leu Arg
                   165                 170                 175
   Ile His Asn Val Gln Lys Glu Asp Ala Gly Gln Tyr Arg Cys Val Ala
               180                 185                 190
   Lys Asn Ser Leu Gly Thr Ala Tyr Ser Lys Leu Val Lys Leu Glu Val
           195                 200                 205
   Glu Val Phe Ala Arg Ile Leu Arg Ala Pro Glu Ser His Asn Val Thr
       210                 215                 220
   Phe Gly Ser Phe Val Thr Leu Arg Cys Thr Ala Ile Gly Ile Pro Val
   225                 230                 235                 240
   Pro Thr Ile Ser Trp Ile Glu Asn Gly Asn Ala Val Ser Ser Gly Ser
                   245                 250                 255
   Ile Gln Glu Ser Val Lys Asp Arg Val Ile Asp Ser Arg Leu Gln Leu
               260                 265                 270
   Phe Ile Thr Lys Pro Gly Leu Tyr Thr Cys Ile Ala Thr Asn Lys His
           275                 280                 285
   Gly Glu Lys Phe Ser Thr Ala Lys Ala Ala Ala Thr Val Ser Ile Ala
       290                 295                 300
   Glu Trp Ser Lys Ser Gln Lys Asp Ser Gln Gly Tyr Cys Ala Gln Tyr
   305                 310                 315                 320
   Arg Gly Glu Val Cys Asp Ala Val Leu Ala Lys Asp Ala Leu Val Phe
                   325                 330                 335
   Phe Asn Thr Ser Tyr Arg Asp Pro Glu Asp Ala Gln Glu Leu Leu Ile
               340                 345                 350
   His Thr Ala Trp Asn Glu Leu Lys Ala Val Ser Pro Leu Cys Arg Pro
           355                 360                 365
   Ala Ala Glu Ala Leu Leu Cys Asn His Leu Phe Gln Glu Cys Ser Pro
       370                 375                 380
   Gly Val Val Pro Thr Pro Met Pro Ile Cys Arg Glu Tyr Cys Leu Ala
   385                 390                 395                 400
   Val Lys Glu Leu Phe Cys Ala Lys Glu Trp Gln Ala Met Glu Gly Lys
                   405                 410                 415
   Ala His Arg Gly Leu Tyr Arg Ser Gly Met His Leu Leu Pro Val Pro
               420                 425                 430
   Glu Cys Ser Lys Leu Pro Ser Met His Arg Asp Pro Thr Ala Cys Thr
           435                 440                 445
   Arg Leu Pro Tyr Leu Gly Asn Lys Glu Val Pro Pro Asp Phe Gly Ser
       450                 455                 460
<210> 5
   <211> 26
   <212> DNA
   <213> Artificial Sequence
<220>
   <223> Description of Artificial Sequence: primer
<400> 5
   gttctccaga aggaacttcg tcctgc 26
<210> 6
   <211> 27
   <212> DNA
   <213> Artificial Sequence
<220>
   <223> Description of Artificial Sequence: primer
<400> 6
   ccgtgcagcg cagtaaatgc catcatc 27
<210> 7
   <211> 20
   <212> DNA
   <213> Artificial Sequence
<220>
   <223> Description of Artificial Sequence: primer
<400> 7
   ggattaatca tgagagagct 20
<210> 8
   <211> 20
   <212> DNA
   <213> Artificial Sequence
<220>
   <223> Description of Artificial Sequence: primer
<400> 8
   gtaaatttct cttaacctcc 20
<210> 9
   <211> 24
   <212> DNA
   <213> Artificial Sequence
<220>
   <223> Description of Artificial Sequence: primer
<400> 9
   tgcacaagac tgccatattt aggt 24
<210> 10
   <211> 23
   <212> DNA
   <213> Artificial Sequence
<220>
   <223> Description of Artificial Sequence: primer
<400> 10
   cgttccttga ctggaaacag taa 23
<210> 11
   <211> 519
   <212> PRT
   <213> Mus musculus
<400> 11
   Met Arg Glu Leu Val Asn Ile Pro Leu Leu Gln Met Leu Thr Leu Val
   1               5                   10                  15
   Ala Phe Ser Gly Thr Glu Lys Leu Pro Lys Ala Pro Val Ile Thr Thr
               20                  25                  30
   Pro Leu Glu Thr Val Asp Ala Leu Val Glu Glu Val Ala Thr Phe Met
           35                  40                  45
   Cys Ala Val Glu Ser Tyr Pro Gln Pro Glu Ile Ser Trp Thr Arg Asn
       50                  55                  60
   Lys Ile Leu Ile Lys Leu Phe Asp Thr Arg Tyr Ser Ile Arg Glu Asn
   65                  70                  75                  80
   Gly Gln Leu Leu Thr Ile Leu Ser Val Glu Asp Ser Asp Asp Gly Ile
                   85                  90                  95
   Tyr Cys Cys Ile Ala Asn Asn Gly Val Gly Gly Ala Val Glu Ser Cys
               100                 105                 110
   Gly Ala Leu Gln Val Lys Met Lys Pro Lys Ile Thr Arg Pro Pro Ile
           115                 120                 125
   Asn Val Lys Ile Ile Glu Gly Leu Lys Ala Val Leu Pro Cys Thr Thr
       130                 135                 140
   Met Gly Asn Pro Lys Pro Ser Val Ser Trp Ile Lys Gly Asp Asn Ala
   145                 150                 155                 160
   Leu Arg Glu Asn Ser Arg Ile Ala Val Leu Glu Ser Gly Ser Leu Arg
                   165                 170                 175
   Ile His Asn Val Gln Lys Glu Asp Ala Gly Gln Tyr Arg Cys Val Ala
               180                 185                 190
   Lys Asn Ser Leu Gly Thr Ala Tyr Ser Lys Leu Val Lys Leu Glu Val
           195                 200                 205
   Glu Glu Asp Arg Glu Pro Glu Gln Asp Ala Lys Val Phe Ala Arg Ile
       210                 215                 220
   Leu Arg Ala Pro Glu Ser His Asn Val Thr Phe Gly Ser Phe Val Thr
   225                 230                 235                 240
   Leu Arg Cys Thr Ala Ile Gly Ile Pro Val Pro Thr Ile Ser Trp Ile
                   245                 250                 255
   Glu Asn Gly Asn Ala Val Ser Ser Gly Ser Ile Gln Glu Ser Val Lys
               260                 265                 270
   Asp Arg Val Ile Asp Ser Arg Leu Gln Leu Phe Ile Thr Lys Pro Gly
           275                 280                 285
   Leu Tyr Thr Cys Ile Ala Thr Asn Lys His Gly Glu Lys Phe Ser Thr
       290                 295                 300
   Ala Lys Ala Ala Ala Thr Val Ser Ile Ala Val Pro Pro Pro Trp Phe
   305                 310                 315                 320
   Ser Met Asp Thr Ser Phe Leu Trp Thr Glu Trp Ser Lys Ser Gln Lys
                   325                 330                 335
   Asp Ser Gln Gly Tyr Cys Ala Gln Tyr Arg Gly Glu Val Cys Asp Ala
               340                 345                 350
   Val Leu Ala Lys Asp Ala Leu Val Phe Phe Asn Thr Ser Tyr Arg Asp
           355                 360                 365
   Pro Glu Asp Ala Gln Glu Leu Leu Ile His Thr Ala Trp Asn Glu Leu
   370 375 380
   Lys Ala Val Ser Pro Leu Cys Arg Pro Ala Ala Glu Ala Leu Leu Cys
   385 390 395 400
   Asn His Leu Phe Gln Glu Cys Ser Pro Gly Val Val Pro Thr Pro Met
   405 410 415
   Pro Ile Cys Arg Glu Tyr Cys Leu Ala Val Lys Glu Leu Phe Cys Ala
   420 425 430
   Lys Glu Trp Gln Ala Met Glu Gly Lys Ala His Arg Gly Leu Tyr Arg
   435 440 445
   Ser Gly Met His Leu Leu Pro Val Pro Glu Cys Ser Lys Leu Pro Ser
   450 455 460
   Met His Arg Asp Pro Thr Ala Cys Thr Arg Leu Pro Tyr Leu Asp Tyr
   465 470 475 480
   Lys Lys Glu Asn Ile Thr Thr Phe Pro Ser Ile Thr Ser Ser Arg Pro
   485 490 495
   Ser Ala Asp Ile Pro Asn Leu Pro Ala Ser Thr Ser Ser Phe Ala Val
   500 505 510
   Ser Pro Ala Tyr Ser Met Thr
   515
<210> 12
   <211> 386
   <212> PRT
   <213> Homo sapiens
<400> 12
   Met Arg Glu Leu Val Asn Ile Pro Leu Val His Ile Leu Thr Leu Val
   1 5 10 15
   Ala Phe Ser Gly Thr Glu Lys Leu Pro Lys Ala Pro Val Ile Thr Thr
   20 25 30
   Pro Leu Glu Thr Val Asp Ala Leu Val Glu Glu Val Ala Thr Phe Met
   35 40 45
   Cys Ala Val Glu Ser Tyr Pro Gln Pro Glu Ile Ser Trp Thr Arg Asn
   50 55 60
   Lys Ile Leu Ile Lys Leu Phe Asp Thr Arg Tyr Ser Ile Arg Glu Asn
   65                  70                  75                  80
   Gly Gln Leu Leu Thr Ile Leu Ser Val Glu Asp Ser Asp Asp Gly Ile
                   85                  90                  95
   Tyr Cys Cys Thr Ala Asn Asn Gly Val Gly Gly Ala Val Glu Ser Cys
               100                 105                 110
   Gly Ala Leu Gln Val Lys Met Lys Pro Lys Ile Thr Arg Pro Pro Ile
           115                 120                 125
   Asn Val Lys Ile Ile Glu Gly Leu Lys Ala Val Leu Pro Cys Thr Thr
       130                 135                 140
   Met Gly Asn Pro Lys Pro Ser Val Ser Trp Ile Lys Gly Asp Ser Pro
   145                 150                 155                 160
   Leu Arg Glu Asn Ser Arg Ile Ala Val Leu Glu Ser Gly Ser Leu Arg
                   165                 170                 175
   Ile His Asn Val Gln Lys Glu Asp Ala Gly Gln Tyr Arg Cys Val Ala
               180                 185                 190
   Lys Asn Ser Leu Gly Thr Ala Tyr Ser Lys Val Val Lys Leu Glu Val
           195                 200                 205
   Glu Glu Glu Ser Glu Pro Glu Gln Asp Thr Lys Val Phe Ala Arg Ile
       210                 215                 220
   Leu Arg Ala Pro Glu Ser His Asn Val Thr Phe Gly Ser Phe Val Thr
   225                 230                 235                 240
   Leu His Cys Thr Ala Thr Gly Ile Pro Val Pro Thr Ile Thr Trp Ile
                   245                 250                 255
   Glu Asn Gly Asn Ala Val Ser Ser Gly Ser Ile Gln Glu Ser Val Lys
               260                 265                 270
   Asp Arg Val Ile Asp Ser Arg Leu Gln Leu Phe Ile Thr Lys Pro Gly
           275                 280                 285
   Leu Tyr Thr Cys Ile Ala Thr Asn Lys His Gly Glu Lys Phe Ser Thr
       290                 295                 300
   Ala Lys Ala Ala Ala Thr Ile Ser Ile Ala Glu Trp Arg Glu Tyr Cys
   305                 310                 315                 320
   Leu Ala Val Lys Glu Leu Phe Cys Ala Lys Glu Trp Leu Val Met Glu
                   325                 330                 335
   Glu Lys Thr His Arg Gly Leu Tyr Arg Ser Glu Met His Leu Leu Ser
               340                 345                 350
   Val Pro Glu Cys Ser Lys Leu Pro Ser Met His Trp Asp Pro Thr Ala
           355                 360                 365
   Cys Ala Arg Leu Pro His Leu Gly Asn Thr Glu Phe Ser Gln Asp Phe
       370                 375                 380
   Gly Gly
   385

## Description

### TECHNICAL FIELD

The present invention relates to a biomarker for the diagnosis of aging or amyotrophy.

### BACKGROUND ART

With the arrival of a super-aged society, a valid biomarker for preventing the state of long-term care needs caused by a decline in locomotion performance and amyotrophy is sought, but no crucial biomarker has been provided. In particular, since when amyotrophy has progressed, it is impossible to treat it, a biomarker for an early diagnosis and treatment is required. Further, a biomarker for incurable neuromuscular diseases leading to amyotrophy is also required in order to develop a valid therapy and a valid therapeutic agent.

However, in the current clinical field or the field of preventive care, a diagnosis is carried out based on the measurements of muscle strength, muscle mass, and exercise performance, as well as a daily activity score by interview. Since these methods have poor efficacy in an early diagnosis prior to the progression of amyotrophy, it is necessary to develop a biomarker capable of preventing or treating amyotrophy, by an early diagnosis of changes in muscles.

For example, amyotrophic lateral sclerosis (ALS) is diagnosed based on characteristic clinical symptoms, and in addition to this, it has been shown that examinations, such as needle electromyography, transcranial magnetic stimulation (TMS), and neuroimaging, are valid for supporting the diagnosis. Further, a biomarker has been considered to be valid for an early diagnosis of ALS, as well as a reduction in the number of subjects in a clinical trial, a period shortening, and an objective judgment of drug efficacy, and therefore, identification has been examined using a cerebrospinal fluid or plasma derived from ALS patients, but there is no biomarker that was demonstrated to be useful in ALS (Non-patent literature 1).

Although an autoantibody is used in the definitive diagnosis of myasthenia gravis, it is not valid for a judgment of the effectiveness or side effects of a therapy, as well as evaluation of severity. Further, single-fiber electromyography is a good indication for diagnosing symptoms at the time of measurement, but is not valid for the prognosis after treatment. Therefore, a biomarker that can be commonly used in an animal model of myasthenia gravis and a patient, and that can be used in objectively evaluating therapeutic effects is required (Non-patent literature 2).

Inflammatory markers such as TNF-α and IL-6 were reported as biomarkers for sarcopenia (Non-patent literatures 3-6). However, since they have a poor specificity, it is difficult to use them for an early diagnosis and treatment of amyotrophy.

### CITATION LIST

### PATENT LITERATURE

[Non-patent literature 1] Costa J, Gomes C, de Carvalho M, CNS Neurol Disord Drug Targets. 2010; 9: 764-778
[Non-patent literature 2] Kaminski H, Kusner L, Wolfe G, et al., Ann N Y Acad Sci 2012; 1275(1): 101-106.
[Non-patent literature 3] Visser M, Pahor M, Taaffe DR, et al., J Gerontol A Biol Sci Med Sci 2002; 57: M326-332.
[Non-patent literature 4] Payette H, Roubenoff R, Jacques PF, et al., J Am Geriatr Soc 2003; 51: 1237-1243.
[Non-patent literature 5] Schaap LA, Pluijm SM, Deeg DJ, et al., Am J Med 2006; 119: 526 e529-517.
[Non-patent literature 6] Schaap, LA, Pluijm SM, Deeg DJ, et al., J Gerontol A Biol Sci Med Sci 2009; 64: 1183-1189.
[Non-patent literature 7] Jorgensen, LH, et al., Am J Pathol, 2007; 171(5): 1599-1607

### SUMMARY OF INVENTION

### TECHNICAL PROBLEM

Under these circumstances, an object of the present invention is to provide a novel biomarker for the diagnosis of aging or amyotrophy, and another object is, based on the biomarker, to provide a method for determining the state of aging or amyotrophy, a method for determining the efficacy of therapeutic or preventive effects on aging or amyotrophy, and a method of screening for a therapeutic or preventive agent for aging or amyotrophy.

### SOLUTION TO PROBLEM

It is known that a receptor-type MuSK (muscle-specific kinase) protein, which is a tyrosine kinase that is expressed in the muscular side (i.e., the postsynaptic membrane) of neuromuscular synapses (neuromuscular junctions), is an essential protein for the formation of neuromuscular synapses, and also plays an important role in the functional maintenance of neuromuscular synapses. The inventor found that a secreted MuSK protein, which was generated by a transcription which passed through a splicing signal at an exon upstream of a membrane binding domain of the receptor-type MuSK protein, was expressed in skeletal muscle, and reported that myasthenia gravis occurred by immunizing a mouse with the secreted MuSK (details will be described below). However, the function of the secreted MuSK in vivo was unknown until now.

The inventor measured the expression level of mRNAs for the secreted MuSK and the receptor-type MuSK in a mouse skeletal muscle (Example 3), and the amount of a MuSK protein which was present in a free state in a mouse serum (Example 4), and confirmed that the expression level of mRNA for the secreted MuSK, and the amount of the MuSK protein in blood increased with age; and that the expression level of mRNA for the secreted MuSK, the expression level of mRNA for the receptor-type MuSK, and the amount of the MuSK protein in blood increased in mice in which morphological and functional defects of neuromuscular synapses were in progress (a motor-nerve-cleaved mouse or a cast-immobilized mouse). Further, in an amyotrophic lateral sclerosis (ALS) mouse model and a myasthenia gravis (MG) mouse model, the inventor confirmed that, in the former (ALS mouse), an increase in the amount of the free MuSK protein contained in a serum was observed from prior to the onset, and a further increase was observed in accordance with the progress of the disease (Example 6); and that, in the latter (MG mouse), an increase in the amount of the free MuSK protein contained in a serum was observed in accordance with the onset (Example 7). From these findings, the inventor has found that the mRNA for the secreted MuSK, the mRNA for the receptor-type MuSK, or the free MuSK protein in blood can be used as a biomarker for aging, as an indicator that shows the function of neuromuscular synapses, or a biomarker that allows an early diagnosis for amyotrophy.

Furthermore, the inventor measured the amounts of the free MuSK protein in sera collected from patients with myasthenia gravis (in which a decline in muscle strength and mild or moderate amyotrophy were caused by an autoantibody against an acetylcholine receptor (a receptor for a transmitter expressed in the muscular side of a neuromuscular synapse)) and patients with amyotrophic lateral sclerosis (in which amyotrophy had highly progressed) (Example 5); confirmed that the amount of the MuSK protein in the blood of the patient with amyotrophic lateral sclerosis (in which neuromuscular synapses disappeared and amyotrophy was caused due to the cell death of motor nerves) was remarkably reduced (substantially disappeared) in comparison with healthy people; and has found that the free MuSK in blood can be used as a biomarker for severe amyotrophy.

Furthermore, the inventor confirmed, from a serum collected from mice with ALS (Example 8) and a culture supernatant of mouse cultured myoblast cell line C2C12 (Example 9), that a free MuSK protein (a truncated MuSK protein) that was cleaved from the receptor-type MuSK protein by a protease and released into a culture supernatant was included in the free MuSK protein. The inventor confirmed that the amount of the free MuSK protein in the culture supernatant of mouse cultured myoblast cell line C2C12 was reduced by adding an ADAM inhibitor GM6001 to the culture medium (Example 10), and has found that a therapeutic or preventive agent for aging or amyotrophy can be screened by measuring the amount of the free MuSK protein or mRNA contained in a sample collected from an animal model, or a culture supernatant of a cell line. The inventor confirmed that the ADAM inhibitor GM6001 exhibited an increasing activity of an acetylcholine receptor (AChR) clustering, which was an indicator of synaptogenesis, against mouse C2C12 myotube cells (Example 11), and confirmed that substances selected by the screening system actually exhibited an activity of promoting synaptogenesis, and therefore, could be candidates for the therapeutic or preventive agent for aging or amyotrophy.

The present invention is based on these findings.

The present invention is defined by the appendent claims. The present disclosure relates to:
[15a] a method for treating or preventing aging or amyotrophy, said method comprising administering to a subject in need thereof, a substance capable of reducing an amount of a free MuSK protein, or an expression level of an mRNA for a secreted MuSK, or a substance capable of increasing acetylcholine receptor (AChR) clustering, in an amount effective therefor;
[15b] use of a substance capable of reducing an amount of a free MuSK protein, or an expression level of an mRNA for a secreted MuSK, or a substance capable of increasing acetylcholine receptor (AChR) clustering, in the manufacture of a therapeutic or preventive agent for aging or amyotrophy; and
[15c] a substance capable of reducing an amount of a free MuSK protein, or an expression level of an mRNA for a secreted MuSK, or a substance capable of increasing acetylcholine receptor (AChR) clustering, for the treatment or prevention of aging or amyotrophy.

The term "amyotrophy" as used herein includes, for example, sarcopenia, myasthenia gravis (including anti-MuSK antibody positive myasthenia gravis, anti-acetylcholine receptor (AChR) antibody positive myasthenia gravis, anti-LRP4 (low-density lipoprotein receptor (LDLR) related protein 4) antibody positive myasthenia gravis, and known autoantibody negative myasthenia gravis), incurable neuromuscular disease, disuse muscle atrophy, motor nerve damage, cachexia, chronic obstructive lung disease, Crohn disease, sarcopenic obesity, arteriosclerosis with amyotrophy, amyotrophy associated with cerebral vascular disease, type I or type II diabetic amyotrophy, muscle damage, and muscle tissue reconstruction after injury or surgery treatment.

Sarcopenia is pathological conditions of a decline in muscle strength leading to a decline in locomotion performance and eventually amyotrophy, due to aging. Although the cause is unknown, the progression of the pathological conditions results in functional and morphological defects in neuromuscular synapses (a junction between a neuron and a muscle). It has been revealed from experiments of aged mice that the structure of neuromuscular synapses is improved by caloric restriction and exercise (Valdez G, Tapia JC, Kang H, et al. PNAS 2010; 107: 14863-14868.).

In myasthenia gravis, a morphological defect in neuromuscular synapses and a decline of neurotransmission occurs due to an autoantibody against a neuromuscular synapse, and a decline in muscle strength and amyotrophy are caused. An acetylcholine receptor (AChR), MuSK, and LRP4 has been found as autoantibodies that cause myasthenia gravis, but unexplained myasthenia gravis still remains.

Incurable neuromuscular diseases include motor neuron diseases (including amyotrophic lateral sclerosis, primary lateral sclerosis, spinal muscular atrophy, and spinal and bulbar muscular atrophy), polymyositis, Guillain-Barré syndrome, congenital myasthenia, muscular dystrophy, distal myopathy, congenital myopathy, glycogen storage disease, mitochondrial myopathy, steroid myopathy, inflammatory myopathy, endocrine myopathy, and lipid storage myopathy. The causes of these diseases in which amyotrophy is developed are different, but in the process leading to amyotrophy, functional and morphological defects in neuromuscular synapses result in an attenuation of neurotransmission, and a decline in muscle strength and amyotrophy become worse.

Disuse muscle atrophy is amyotrophy accompanied by functional and morphological defects in synapses, caused by a long-term treatment on a sick bed or cast immobilization. It was revealed that neuroskeletal synapses were maintained by reciprocal transmission signals from muscle and motor neuron terminals (Yumoto N, Kim N, Burden SJ. Nature 2012;489:438-442.). In healthy people, plasticity of synapses is maintained, and the function and structure of synapses can be restored by the regeneration ability of synapses. It was revealed, using healthy rats, that a decline in synaptic structure was induced by insufficient exercise (Deschenes MR, Tenny KA, Wilson MH. Neuroscience 2006;137:1277-1283.).

The term "mild or moderate amyotrophy" as used herein means the state in which a functional defect of neuroskeletal synapses (for example, a decline in signal transmission) is observed, but the reactivity of skeletal muscle (for example, regeneration or maintenance of neuromuscular synapses) is maintained.

The term "severe amyotrophy" as used herein means the state in which the function of neuroskeletal synapses is lost, and the reactivity of skeletal muscle is lost.

### ADVANTAGEOUS EFFECTS OF INVENTION

According to the present invention, a novel biomarker for the diagnosis of aging or amyotrophy can be provided.

According to the biomarker of the present invention, a method for determining the state of aging or amyotrophy, a method for determining the efficacy of therapeutic or preventive effects (for example, a medication therapy, an exercise therapy, or a nutritional therapy) on aging or amyotrophy, and a method of screening for a therapeutic or preventive agent for aging or amyotrophy.

### BRIEF DESCRIPTION OF THE DRAWINGS

[Fig. 1] Fig. 1 is a graph showing the results obtained by measuring the mRNA expression levels of a secreted MuSK and a receptor-type MuSK in mouse skeletal muscles of 6-month-old C57BL/6 mice, 6-month-old mice after 11 days from cast immobilization, 6-month-old mice after 11 days from cleavage of the sciatic nerve, and 29-month-old elderly mice.
[Fig. 2] Fig. 2 is a graph showing the results obtained by measuring the amount of a free MuSK protein contained in mouse sera of 8-month-old mice (control), 20-month-old aged mice, 27-month-old aged mice, 32-month-old aged mice, and 6-month-old mice after 11 days from cleavage of the sciatic nerve.
[Fig. 3] Fig. 3 is a graph showing the results obtained by measuring the amount of a free MuSK protein contained in patient sera collected from patients with amyotrophic lateral sclerosis (ALS, N = 8) and patients with myasthenia gravis (MG, N = 3).
[Fig. 4] Fig. 4 is a graph showing the results obtained by measuring the amount of a free MuSK protein contained in sera of wild-type mice (control) and ALS mouse models (G93A-SOD1), prior to onset (4-week-old, 6-week-old, 8-week-old, and 10-week-old).
[Fig. 5] Fig. 5 is a graph showing the results obtained by measuring the amount of a free MuSK protein contained in sera of wild-type mice (control) and ALS mouse models (G93A-SOD1), at 10-week-old, 14-week-old, and 18-week-old, and at the time of death.
[Fig. 6] Fig. 6 is a graph showing the results obtained by measuring the amount of a free MuSK protein contained in sera of wild-type mice (control) and myasthenia gravis (MG) mouse models (EAMG), at 6-week-old to 8-week-old.
[Fig. 7] Fig. 7 is a photograph, in place of drawings, showing the results of Western blotting (rabbit anti-mouse MuSK monoclonal antibody) of an immunoprecipitate obtained by subjecting a serum collected from ALS mouse models to ammonium sulfate fractionation, followed by carrying out immunoprecipitation using mouse anti-MuSK extracellular domain monoclonal antibodies.
[Fig. 8] Fig. 8 is a photograph, in place of drawings, showing the results of Western blotting (rabbit anti-mouse MuSK monoclonal antibody) of a purified fraction obtained by subjecting a culture supernatant of a mouse cultured myoblast cell line C2C12 to ammonium sulfate fractionation, followed by carrying out purification using an anti-mouse MuSK antibody column.
[Fig. 9] Fig. 9 is a photograph, in place of drawings, showing the results of CBB staining of the purified fraction obtained by subjecting a culture supernatant of a mouse cultured myoblast cell line C2C12 to ammonium sulfate fractionation, followed by carrying out purification using an anti-mouse MuSK antibody column.
[Fig. 10] Fig. 10 schematically shows the results of a comparison of amino acid sequences obtained by analyzing Bands 1-5 (a region of 85 kDa to 50 kDa) shown in Fig. 9 by LC-MS/MS, with the amino acid sequence (SEQ ID NO: 11) of an extracellular domain of a mouse receptor-type MuSK protein.
[Fig. 11] Fig. 11 is a graph showing the results obtained by measuring the amount of a free MuSK protein contained in a culture supernatant, after culturing mouse cultured myoblast cell line C2C12 in the presence of an ADAM inhibitor GM6001.
[Fig. 12] Fig. 12 is a graph showing the results obtained by culturing mouse cultured myoblast cell line C2C12 in the presence of an ADAM inhibitor GM6001, followed by carrying out acetylcholine receptor (AChR) clustering staining.

### DESCRIPTION OF EMBODIMENTS

### (1) Biomarker, and method of determining the state of aging or amyotrophy, and method of determining the efficacy of therapeutic or preventive effects on aging or amyotrophy of the present invention

A secreted MuSK (muscle-specific kinase) protein, or a gene product (mRNA) thereof is used as a biomarker in the present invention. The MuSK protein includes a receptor-type MuSK protein, of which the function has been well analyzed, and a secreted MuSK protein, of which the function in the living body is unknown. The secreted MuSK protein, or a MuSK protein which is present in a free state in body fluids, such as blood, is used in the present invention.

The receptor-type MuSK protein is a tyrosine kinase, which is expressed in the muscular side (i.e., the postsynaptic membrane) of neuromuscular synapses (Valenzuela DM, Stitt TN, DiStefano PS, et al. Neuron 1995; 15: 573-84., and Ganju P, Walls E, Brennan J, Reith AD. Oncogene 1995; 11: 281-90.), and a functional protein essential for the formation of neuromuscular synapses (DeChiara TM, Bowen DC, Valenzuela DM, et al. Cell 1996; 85: 501-12.). Agrin, which is a heparin sulfate proteoglycan released from motor nerve terminals, binds to Lrp4 (low-density lipoprotein receptor (LDLR) related protein 4), which is expressed in the postsynaptic membrane, to activate MuSK (Zhang B, Luo S, Wang Q, Suzuki T, Xiong WC, Mei L. Neuron 2008; 60: 285-97.). The agrin-Lrp4-MuSK signaling plays an important role in the maintenance of neuromuscular synapses.

On the other hand, the secreted MuSK protein is a tyrosine kinase, which is generated by a transcription which passes through a splicing signal at an exon upstream of its membrane binding domain. The cDNA sequence (SEQ ID NO: 3) and the amino acid sequence (SEQ ID NO: 4) of mouse secreted MuSK were reported by the present inventor and his coworkers (GenBank: AY360453.1), but the cDNA sequence (SEQ ID NO: 1) and the amino acid sequences (SEQ ID NOS: 2 and 12) of human secreted MuSK have now been found for the first time. Although the function of the secreted MusK in the living body is unknown, the present inventor and his coworkers reported that myasthenia gravis is caused by immunizing a mouse with mouse secreted MuSK (Shigemoto K, Kubo S, Maruyama N, et al. J Clin Invest 2006; 116: 1016-24.).

The term "secreted MuSK protein" as used herein means, in a narrow sense, a non-receptor-type MuSK protein that is generated by a transcription which passes through a splicing signal at an exon upstream of the membrane binding domain, and means, in a broad sense, a MuSK protein that is present in a free state in body fluids (for example, blood, cerebrospinal fluid, lymph fluid, urine, ascites, pleural effusion, or the like, in particular, blood). Examples of the MuSK protein that is present in a free state in body fluids include the secreted MuSK protein (in a narrow sense), and a protein (a truncated MuSK protein) consisting of an extracellular domain portion (or its partial fragment), which is generated by cleaving the receptor-type MuSK protein into the extracellular domain and the membrane binding domain, by a mechanism in the living body (for example, by enzymatic cleavage in vivo). In the present invention, the secreted MuSK protein (in a narrow sense) can be used as the biomarker in an embodiment, and the secreted MuSK protein (in a narrow sense) can be used as the biomarker in another embodiment. The secreted MuSK protein (in a narrow sense) is sometimes and simply referred to as "the secreted MuSK protein". The secreted MuSK protein (in a broad sense) is sometimes referred to as "the MuSK protein that is present in a free state", or sometimes and simply referred to as "the free MuSK protein".

The secreted MuSK protein (in a narrow sense) can be analyzed using an antibody that recognizes a partial sequence at the C-terminal side specific to the secreted MuSK protein (for example, amino acids 452-464 of SEQ ID NO: 2 (human), amino acids 374-386 of SEQ ID NO: 12 (human), or amino acids 452-464 of SEQ ID NO: 4 (mouse)).

The secreted MuSK protein (in a broad sense) can be analyzed using an antibody that recognizes a sequence common to the receptor-type MuSK protein and the secreted MuSK protein (for example, amino acids 1-451 of SEQ ID NO: 2 (human), amino acids 1-373 of SEQ ID NO: 12 (human), or amino acids 1-451 of SEQ ID NO: 4 (mouse)), in the extracellular domain.

The biomarker of the present invention can be used as a biomarker for determining the state of aging or amyotrophy. The "determination of the state of aging or amyotrophy" as used herein includes, for example, a determination of the degree of aging, an early diagnosis of amyotrophy, a detection of severe amyotrophy, a measurement of the produced amount of an autoantigen (secreted MuSK or receptor-type MuSK, particularly secreted MuSK), a differentiation of a specific disease (for example, a differentiation between amyotrophic lateral sclerosis and myasthenia gravis), and the like.

In the present invention, as shown in Example 3 described below, receptor-type MuSK mRNA and secreted MuSK mRNA are expressed in skeletal muscle. Whereas the mRNA expression of the receptor-type MuSK is not affected by aging, the mRNA expression of the secreted MuSK increases due to aging, and therefore, the secreted MuSK mRNA can be used as a biomarker for aging. Further, as shown in the same Example, in the early stage of amyotrophy caused by nerve cleavage or cast immobilization, both the receptor-type MuSK mRNA and the secreted MuSK mRNA increase in skeletal muscle, and therefore, these mRNAs can be used as a biomarker for an early diagnosis for amyotrophy.

As shown in Example 4, the secreted MuSK protein, i.e., the free MuSK, is expressed in blood, and the amount of the secreted MuSK protein increases due to aging, and therefore, the secreted MuSK protein can be used as a biomarker for aging. Further, as shown in the same Example, in the early stage of amyotrophy caused by nerve cleavage, the amount of the secreted MuSK protein increase in blood, and therefore, the secreted MuSK protein can be used as a biomarker for an early diagnosis for amyotrophy.

That is to say, in the present invention, when the amount of the secreted MuSK protein, i.e., the amount of the free MuSK protein, or the expression level of the mRNA for the secreted MuSK (i.e., the mRNA for non-receptor-type MuSK; hereinafter the same unless otherwise specified) shows a high value, in comparison with healthy people or healthy conditions, it can be judged that aging is in progress, that functional defects in neuromuscular synapses are in progress, and that amyotrophy is in progress.

Further, when, after a prevention or treatment for aging or amyotrophy (for example, a medication therapy, an exercise therapy, or a nutritional therapy) is carried out, the amount of the secreted MuSK protein, i.e., the amount of the free MuSK protein, or the expression level of the mRNA for the secreted MuSK is reduced, in comparison with the same before the prevention or treatment, it can be judged that its therapeutic or preventive effects are valid (in the case of a medication therapy, the drug is valid.)

More particularly, with respect to a subject suspected of the progression of amyotrophy or the like, the average value and the threshold are determined in advance from measured values obtained from a population of healthy people showing similar conditions (for example, race, sex, age, physique (in particular, skeletal muscle mass), exercise history, medical history, or the like) to the subject, and the measured value of the subject is compared with the threshold to carry out the judgment.

Alternatively, with respect to a subject suspected of the progression of amyotrophy or the like, measured values in healthy conditions at the adolescent stage to the middle-adult stage or at arbitrary stages (or the early stage of amyotrophy or the like) of the subject are obtained in advance, and further, measured values are regularly and repeatedly obtained at predetermined intervals (for example, at intervals of two weeks to one year), and are compared with the measured values in healthy conditions to carry out the judgment.

For example, with respect to the average value of healthy people, or with respect to the measured values of the subject in healthy conditions (or at the early stage of amyotrophy or the like), when the measured value of the subject at the judgment is, for example, at least 1.2 times higher, at least 1.4 times higher in an embodiment, and at least 1.5 times higher in another embodiment, it can be judged that the measured value is high.

Further, in the present invention, the secreted MuSK protein, i.e., the free MuSK protein, or the secreted MuSK mRNA can be used as a biomarker for severe amyotrophy (for example, amyotrophic lateral sclerosis, in particular, the end stage of onset in a human).

More particularly, when the amount of the secreted MuSK protein, i.e., the amount of the free MuSK protein, or the expression level of the mRNA for the secreted MuSK shows a remarkably low value, in comparison with healthy people or healthy conditions (or the early stage of amyotrophy or the like), or does not increase at all even when the measurements are repeatedly carried out at predetermined intervals (for example, at intervals of two weeks to one year), it can be judged that it has progressed to severe amyotrophy.

Further, when, after a prevention (prevention of progression) or treatment for severe amyotrophy is carried out, the amount of the secreted MuSK protein, i.e., the amount of the free MuSK protein, or the expression level of the mRNA for the secreted MuSK increases, in comparison with the same before the prevention or treatment, it can be judged that its therapeutic or preventive effects are valid (in the case of a medication therapy, the drug is valid.).

For example, with respect to the average value of healthy people, or with respect to the measured values of the subject in healthy conditions (or at the early stage of amyotrophy or the like), when the measured value of the subject at the judgment is, for example, 1/2 or less, 1/4 or less in an embodiment, 1/6 or less in another embodiment, and 1/10 or less in still another embodiment, it can be judged that the measured value is remarkably low.

Alternatively, when the concentration of the secreted MuSK protein (i.e., the free MuSK protein) in a human serum is, for example, 1 ng/mL or less, 0.5 ng/mL or less in an embodiment, 0.1 ng/mL or less in another embodiment, and 0.05 ng/mL or less in still another embodiment, it can be judged that the measured value is remarkably low.

Further, in the present invention, the amount of an autoantigen produced in blood or skeletal muscle in anti-MuSK antibody positive myasthenia gravis can be determined by measuring the secreted MuSK protein, i.e., the free MuSK protein, or the secreted MuSK mRNA. With respect to a patient with anti-MuSK antibody positive myasthenia gravis, the efficacy of a therapy can be judged by detecting the amount of a produced autoantigen. Further, with respect to a subject with a high risk of developing future anti-MuSK antibody positive myasthenia gravis, or a patient with other autoimmune diseases, the efficacy of a preventive or therapeutic method for anti-MuSK antibody positive myasthenia gravis can be judged by measuring the expression amount of MuSK in blood or skeletal muscle.

Further, in the present invention, the secreted MuSK protein, i.e., the free MuSK protein, or the secreted MuSK mRNA can be used as a biomarker for a differentiation between amyotrophic lateral sclerosis and myasthenia gravis.

More particularly, when the amount of the secreted MuSK protein, i.e., the amount of the free MuSK protein, or the expression level of the mRNA for the secreted MuSK shows a remarkably low value, in comparison with healthy people or healthy conditions, it can be judged to be amyotrophic lateral sclerosis, and when it shows a remarkably high value, it can be judged to be myasthenia gravis (in particular, anti-acetylcholine receptor (AChR) antibody positive myasthenia gravis).

The amount of the secreted MuSK protein (i.e., the free MuSK protein) in blood can be measured by a known protein analysis method, for example, an immunological analysis method using an antibody (for example, an ELISA method, a latex agglutination method, or Western blotting), a biochemical method, such as electrophoresis, a mass spectrometry method, or the like, and an automatic analyzer for clinical testing can be used. As a blood sample, for example, a serum, plasma, whole blood, or the like, may be used.

The amount of the secreted MuSK mRNA in skeletal muscle can be measured by a known mRNA analysis method, for example, a PCR method using PCR primers (for example, a real-time PCR method, or an RT-PCR method), a hybridization method using a nucleic acid probe (for example, Northern blotting), or the like. As a skeletal muscle sample, for example, a muscle biopsy sample, or the like may be used.

As described above, the present invention is based on novel findings, which are contradictory at first glance, that the amount of the secreted MuSK protein (i.e., the amount of the free MuSK protein), or the expression level of the mRNA for the secreted MuSK increases at the early stage of amyotrophy, or at the mild or moderate stage thereof, in comparison with healthy people or healthy conditions (in particular, the adolescent stage to the middle-adult stage) or prior to the onset of amyotrophy, or the like, whereas the amount of the secreted MuSK protein or the mRNA expression level is remarkably reduced (substantially disappeared) at a stage in which amyotrophy has severely progressed (for example, amyotrophic lateral sclerosis, in particular, the end stage of onset in a human), in comparison with healthy people or healthy conditions, or the level at the early stage of amyotrophy, or the like.

With respect to the reason why the secreted MuSK protein, i.e., the free MuSK protein, can be used as a biomarker for an early diagnosis of the function of neuromuscular synapses, or amyotrophy, or as a biomarker for a diagnosis of severe amyotrophy, the inventor has considered the following mechanism of action. Whereas the receptor-type MuSK protein is essential for the formation and the functional maintenance of the muscular side (i.e., the postsynaptic membrane) of neuromuscular synapses, the inventor assumes that the secreted MuSK protein plays a role in the formation and the functional maintenance of the neural side (i.e., the presynaptic membrane) of neuromuscular synapses, as a retrograde signal. According to the assumption, it is considered that, even in healthy conditions, a required amount of the secreted MuSK protein is expressed (constitutive expression) in order to maintain the function of neuroskeletal synapses, depending on the skeletal muscle mass. On the other hand, it is considered that, when functional defects in neuromuscular synapses occur, the secreted MuSK protein increases (compensatory, induced expression), depending on the extent of non-activity of neuromuscular synapses, in order to compensate for the amount and the function of skeletal muscle. That is to say, it is considered that, since the expression amount of the secreted MuSK protein, as a retrograde signal, increases in order to maintain the function of neuromuscular synapses, at the time of progression of amyotrophy, it can be used as a biomarker for aging, and as a biomarker for an early diagnosis of amyotrophy. Further, at a stage in which motor neurons disappear and amyotrophy has severely progressed (for example, amyotrophic lateral sclerosis), unlike the case of myasthenia gravis, it is considered that the secreted MuSK protein in blood is remarkably reduced, because both constitutive expression and compensatory induced expression disappear.

In connection with this, the above mechanism of action is an assumption of the inventor at the present stage, and the present invention is not limited to the mechanism of action.

### (2) Screening method for therapeutic or preventive agent for aging or amyotrophy

The biomarker of the present invention can be used in screening for a therapeutic or preventive agent for aging or amyotrophy.

The screening method of the present invention comprises the steps of:
administering a test substance to an animal model of aging or amyotrophy, or a cell line of the animal model (an administration step);
measuring the amount of a secreted MuSK protein (i.e., the amount of a free MuSK protein), or the expression level of an mRNA for the secreted MuSK protein, in a sample obtained from the animal model, or a culture supernatant of the cell line (a measurement step); and
selecting a candidate (a selection step). If desired, the screening method of the present invention can further comprise the step of confirming the therapeutic or preventive effects of the selected candidate on aging or amyotrophy, using another evaluation system (a confirmation step).

The screening method of the present invention can comprise, instead of the measuring step, the step of analyzing acetylcholine receptor (AChR) clustering in the cell line after the administration of a test substance (an analysis step). In connection with this, both the measuring step and the analysis step can be carried out to select a candidate by comprehensive judgment, in the screening method of the present invention.

Further, the analysis step can be used as an evaluation system in the confirmation step.

As the animal model used in the administration step, for example, a naturally aged animal model, an artificially aged animal model, or an animal model in which amyotrophy is developed, can be used.

Examples of these animal models include sarcopenia, myasthenia gravis (including anti-MuSK antibody positive myasthenia gravis, anti-acetylcholine receptor (AChR) antibody positive myasthenia gravis, anti-LRP4 (low-density lipoprotein receptor (LDLR) related protein 4) antibody positive myasthenia gravis, and known autoantibody negative myasthenia gravis), incurable neuromuscular disease (motor neuron diseases (including amyotrophic lateral sclerosis, primary lateral sclerosis, spinal muscular atrophy, and spinal and bulbar muscular atrophy), polymyositis, Guillain-Barré syndrome, congenital myasthenia, muscular dystrophy, distal myopathy, congenital myopathy, glycogen storage disease, mitochondrial myopathy, steroid myopathy, inflammatory myopathy, endocrine myopathy, and lipid storage myopathy), disuse muscle atrophy, motor nerve damage, cachexia, chronic obstructive lung disease, Crohn disease, sarcopenic obesity, arteriosclerosis with amyotrophy, amyotrophy associated with cerebral vascular disease, type I or type II diabetic amyotrophy, muscle damage, and muscle tissue reconstruction after injury or surgery treatment.

As the cell line of an animal model used in the administration step, for example, a primary cultured cell isolated from the animal model, a passaged cultured cell obtained by passaging the primary cultured cell, an established cultured cell obtained by immortalization, or the like can be used. In particular, when the cell line is an immortalized muscle stem cell or myoblast, it is unnecessary to isolate a muscle stem cell or myoblast from an animal model for each experiment, and therefore, the screening method of the present invention can be efficiently carried out.

The muscle stem cell (satellite cell) can be differentiated into a myoblast, and can be further differentiated into a myotube cell (muscle fiber cell). The muscle stem cell and the myoblast can proliferate, and the myotube cell (muscle fiber cell) is a polykaryocyte generated by a fusion of many myoblasts.

The immortalized muscle stem cell can be prepared, for example, by mating a mouse model with an immortmouse having an IFN-γ-induced H-2Kb-promoter and a temperature-sensitive SV40 large T antigen. In the immortmouse, the SV40 large T antigen is expressed by IFN-γ, and cell immortalization is induced.

More particularly, a mouse model is mated with an immortmouse to obtain offspring, and muscle stem cells are separated from the offspring. The separated muscle stem cells are cultured at 33°C in the presence of 10 ng/mL of IFN-γ to decompose the temperature-sensitive SV40 large T antigen, and the immortalized muscle stem cell can be obtained.

The induction of differentiation from the muscle stem cell to the myoblast and the muscle myotube cell is not particularly limited, but the differentiation can be induced, for example, as shown in the Examples described below. More particularly, the muscle stem cells are subcultured in a cell culture medium supplemented with 10% fetal calf serum (FCS), and the differentiation into the myoblasts is induced by culturing the cells in a medium supplemented with 2.5% horse serum (HS), instead of FCS, at the time of differentiation induction. Further, the myoblasts can be induced to the differentiation into the myotube cells (muscle fiber cells) by subculturing them under the same conditions.

As cell lines which can be used in the present invention, examples of cell lines capable of inducing the differentiation from the myoblast to the myotube cell include a mouse-derived C2C12 myoblast cell line, and a rat-derived L6 myoblast cell line. Further, primary cultured myoblasts, which are collected from skeletal muscle of a patient, or a myoblast cell line obtained by differentiation induction from mesenchymal stem cells, ES cells, or iPS cells can be used.

In the aged animal model, the animal model in which mild or moderate amyotrophy is developed, or the cell lines thereof, among the above-mentioned animal models, it is expected that the amount of the secreted MuSK protein, i.e., the amount of the free MuSK protein, or the expression level of the mRNA for the secreted MuSK increases, and that acetylcholine receptor (AChR) clustering is reduced. In the measurement step and the selection step, the amount of the secreted MuSK protein, i.e., the amount of the free MuSK protein, or the expression level of the mRNA for the secreted MuSK contained in a sample collected from an animal model to which a test substance is administered, or a culture supernatant of a cell line is measured, and the measured value is compared with the value before the administration of the test substance (or a control to which the test substance is not administered) to select, as a candidate for a therapeutic or preventive agent for aging or amyotrophy, a test substance in which the amount of the secreted MuSK protein, i.e., the amount of the free MuSK protein, or the expression level of the mRNA for the secreted MuSK is reduced. In the analysis step and the selection step, AChR clustering is analyzed in a cell line to which a test substance is administered, and the AChR clustering is compared with the clustering before the administration of the test substance (or a control to which the test substance is not administered) to select, as a candidate for a therapeutic or preventive agent for aging or amyotrophy, a test substance in which the AChR clustering increases.

With respect to the selected candidate, the therapeutic or preventive effects on aging or amyotrophy is confirmed using another evaluation system, for example, an animal model which is not used in the administration step among the above-mentioned animal models, or an evaluation system close to humans, in the confirmation step, which can be optionally carried out, and then, a promising candidate is subjected to the next development stage.

As the test substances, which are subjected to the screening method of the present invention, for example, various known compounds (including peptides) registered in chemical files, compounds obtained by combinatorial chemistry techniques (Tetrahedron, 51, 8135-8137, 1995), or random peptides prepared by applying a phage display method (J. Mol. Biol., 222, 301-310, 1991), or the like, can be used. Further, culture supernatants of microorganisms, natural components derived from plants or marine organisms, animal tissue extracts, or the like, can be used as test samples for screening. Furthermore, compounds (including peptides) obtained by chemically or biologically modifying compounds (including peptides) selected by the screening method of the present invention, can be used.

### (3) Pharmaceutical composition for treating or preventing aging or amyotrophy

A candidate selected by the screening method of the present invention, i.e., a substance capable of reducing the amount of a free MuSK protein, or the expression level of an mRNA for secreted MuSK, or a substance capable of increasing acetylcholine receptor (AChR) clustering, can be used as the active ingredient of a pharmaceutical composition for treating or preventing aging or amyotrophy (a therapeutic or preventive agent).

Examples of the substance capable of reducing the amount of the free MuSK protein, or the expression level of the mRNA for secreted MuSK, as the active ingredient of the pharmaceutical composition for treatment or prevention, include a substance of inhibiting the cleavage of the receptor-type MuSK protein, a substance of reducing the expression level of mRNA for secreted MuSK, or a substance of inhibiting the activation of a protease against the receptor-type MuSK protein, or an increase in the expression level thereof, regardless of whether or not it is a substance selected by the screening method of the present invention.

The active ingredient of the pharmaceutical composition for treatment or prevention is preferably a substance of reducing the amount of the free MuSK protein, or the expression level of the mRNA for the secreted MuSK, and increasing acetylcholine receptor (AChR) clustering.

Examples of the substance of inhibiting the cleavage of the receptor-type MuSK protein include an inhibitor against ADAM (a disintegrin and metalloproteinase)(for example, ADAM 12 or ADAM 19, in particular, ADAM 12).

Examples of the inhibitor against ADAM include GM6001, TAPI-1 {N-(R)-(2-(Hydroxyaminocarbonyl)methyl)-4-Methylpentanoyl-L-Nal-L-Alanine2-Aminoethyl amide(L-Nal:L-3-(2'-Naphthyl)alanine)}, TAPI-2{N-(R)-(2-(Hydroxyaminocarbonyl)methyl)-4-Methylpentanoyl-L-t-Butyl-Glycyl-L-Alanine2-Aminoethyl amide}, or the like.

Examples of the substance of reducing the expression level of the mRNA for the secreted MuSK include epigenetically-controlling agents, such as siRNA, miRNA, a DNA methylation enzyme, a histone deacetylase inhibitor, or the like.

Examples of the substance of inhibiting the activation of a protease against the receptor-type MuSK protein, or an increase in the expression level thereof include a substance of reducing the expression level of the protease, or a substance of reducing the enzyme activity of the protease.

The substance of increasing acetylcholine receptor (AChR) clustering can be obtained by the screening method of the present invention using AChR clustering as an indicator.

The formulation of the pharmaceutical composition is not particularly limited, but may be, for example, oral medicines, such as powders, fine particles, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method using, for example, fillers, binders, disintegrating agents, surfactants, lubricants, flowability-enhancers, diluting agents, preservatives, coloring agents, perfumes, tasting agents, stabilizers, humectants, antiseptics, antioxidants or the like, such as gelatin, sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate.

The parenteral administration may be, for example, an injection such as a subcutaneous or intravenous injection, or a per rectum administration. Of the parenteral formulations, an injection is preferably used.

When the injections are prepared, for example, water-soluble solvents, such as physiological saline or Ringer's solution, water-insoluble solvents, such as plant oil or fatty acid ester, agents for rendering isotonic, such as glucose or sodium chloride, solubilizing agents, stabilizing agents, antiseptics, suspending agents, or emulsifying agents may be optionally used, in addition to the active ingredient.

The pharmaceutical composition may be administered in the form of a sustained release preparation using sustained release polymers. For example, the pharmaceutical composition of the present invention may be incorporated into a pellet made of ethylene vinyl acetate polymers, and the pellet may be surgically implanted in a tissue to be treated or prevented.

The pharmaceutical composition may contain the active ingredient in an amount of, but is by no means limited to, 0.01 to 99% by weight, preferably 0.1 to 80% by weight.

A dose of the pharmaceutical composition is not particularly limited, but may be determined dependent upon, for example, the kind of active ingredient, the kind of disease, the age, sex, body weight, or symptoms of the subject, a method of administration, or the like. The pharmaceutical composition may be orally or parenterally administered.

The pharmaceutical composition may be administered as a medicament or in various forms, for example, eatable or drinkable products, such as functional foods or health foods (including drinks), or feeds.

### (4) Protein and polynucleotide of the present invention

The secreted MuSK protein, which is the protein of the present invention, includes:
(a) a protein comprising the amino acid sequence of SEQ ID NO: 2 or SEQ ID NO: 12 (preferably a protein consisting of the amino acid sequence of SEQ ID NO: 2 or SEQ ID NO: 12); and
(b) a protein consisting of an amino acid sequence that has at least 80% identity to the amino acid sequence of SEQ ID NO: 2 or SEQ ID NO: 12, and having secreted MuSK protein activity (hereinafter referred to as a "homologous polypeptide").

The number of amino acids capable of being deleted, substituted, and/or added in the "variation functionally equivalent" is 1 to several amino acids, preferably 1 to 10, more preferably 1 to 7, and most preferably 1 to 5.

The "secreted MuSK protein activity" as used herein means an activity of forming and functionally maintaining the neural side (i.e., the presynaptic membrane) of the neuromuscular synapse as a retrograde signal.

The "homologous polypeptide" is a "protein comprising an amino acid sequence that has at least 80% identity to the amino acid sequence of SEQ ID NO: 2 or SEQ ID NO: 12, and having secreted MuSK protein activity", and a polypeptide comprising an amino acid sequence that has preferably at least 90% identity, more preferably at least 92% identity, still more preferably at least 94% identity, still more preferably at least 95% identity, still more preferably at least 98% identity, and most preferably at least 99% identity, is preferable.

The term "identity" as used herein means the value "Identity" obtained by a NEEDLE program (J Mol Biol 1970; 48: 443-453) search, using the following default parameters:
Gap penalty = 10
Extend penalty = 0.5
Matrix = EBLOSUM62

The polynucleotide of the present invention is not particularly limited, so long as it encodes the protein of the present invention. The polynucleotide may be the full length of genomic DNA, or a polynucleotide consisting of only the translated region, such as mRNA or cDNA.

The vector of the present invention is not particularly limited, so long as it contains the polynucleotide of the present invention. The vector can be prepared by incorporating the polynucleotide into an appropriate vector capable of transforming eukaryotic or prokaryotic host cells. The vector can contain a sequence necessary for the expression of the polypeptide, for example, a promoter or an enhancer, and can further contain a sequence necessary for the confirmation of the introduction into the host, for example, a drug resistance gene.

The transformed cell of the present invention can be prepared by transforming appropriate host cells, such as eukaryotic or prokaryotic host cells, with the vector of the present invention. The transformed cell of the present invention can be used in preparing the polypeptide of the present invention.

### EXAMPLES

The present invention now will be further illustrated by, but is by no means limited to, the following Examples.

### «Example 1: Cloning of mouse and human secreted MuSK genes»

A lambda ZapII vector (Stratagene) was used to construct a cDNA library derived from mRNAs of mouse cultured myoblast cell line C2C12. A probe for human MuSK cDNA gene, which was used in screening, was amplified from human muscle-derived total RNA (Stratagene) by RT-PCR using the following primers:
5'-GTTCTCCAGAAGGAACTTCGTCCTGC-3' (SEQ ID NO: 5)
5'-CCGTGCAGCGCAGTAAATGCCATCATC-3' (SEQ ID NO: 6)

The obtained probe for human MuSK cDNA gene was labeled with an isotope, and multiple mouse MuSK cDNAs were isolated from the previously constructed cDNA library to analyze the gene sequences. The isolated MuSK cDNAs included mouse receptor-type MuSK cDNA having an exon containing a membrane binding domain and an exon containing an intracellular region encoding a tyrosine kinase, and a mouse MuSK cDNA clone in which its translational frame ended at the stop codon without splicing before an exon containing a membrane binding region (mouse secreted MuSK cDNA: SEQ ID NO: 3).

Further human genomic sequence was analyzed, and it was predicted that the same splicing isoform was present. Human secreted MuSK cDNA (SEQ ID NO: 1) was cloned by amplifying it from human muscle-derived mRNAs by RT-PCR using the following primers:
5'-GGATTAATCATGAGAGAGCT-3' (SEQ ID NO: 7)
5'-GTAAATTTCTCTTAACCTCC-3' (SEQ ID NO: 8)

### «Example 2: Expression of secreted MuSK protein»

The cloned mouse and human secreted MuSK genes were inserted into an expression vector pCDNA3.1-myc-his, and the gene constructs were introduced into 293T cells using Fugene 6 (Roche). Culture supernatants after two days from the introduction were electrophoresed on an 8% SDS acrylamide gel, and transferred to a PVDF membrane. An anti-myc antibody was used as a first antibody, and the secreted MuSK proteins were detected using a second antibody labeled with horseradish peroxidase (Pierce).

The human secreted MuSK protein (SEQ ID NO: 2) was detected at the positions of 68 kDa and 66 kDa, and the mouse secreted MuSK protein (SEQ ID NO: 4) was detected at the position of 58 kDa.

### «Example 3: Expression level analysis of secreted and receptor-type MuSK genes in mouse soleus muscles by real-time PCR»

Soleus muscles from 6-month-old C57BL/6 mice, soleus muscles after 11 days from cast immobilization on both sides of the same age mice in the extended position, soleus muscles after 11 days from cleavage of the sciatic nerve of the same age mice, and soleus muscles from 29-month-old elderly mice were collected, and mRNAs were extracted and purified. Comparative determination of the secreted MuSK and the receptor-type MuSK was carried out by real-time PCR using a Taqman method (ABI).

As the specific sequence primer for the secreted MuSK,
5'-side sequence: 5'-TGCACAAGACTGCCATATTTAGGT-3' (SEQ ID NO: 9) was used, and a Taqman probe (custom order, ABI) of
3'-side sequence: 5'-CGTTCCTTGACTGGAAACAGTAA-3' (SEQ ID NO: 10) was used.

For the extracellular domain of the receptor-type MuSK, a Taqman probe (Mm00448006_m1, ABI), which corresponds to the fourth and fifth exons, was used, and a Taqman primer (Mm01346926_ml), which corresponds to the thirteenth and fourteenth exons of the intracellular domain of the receptor-type, was used.

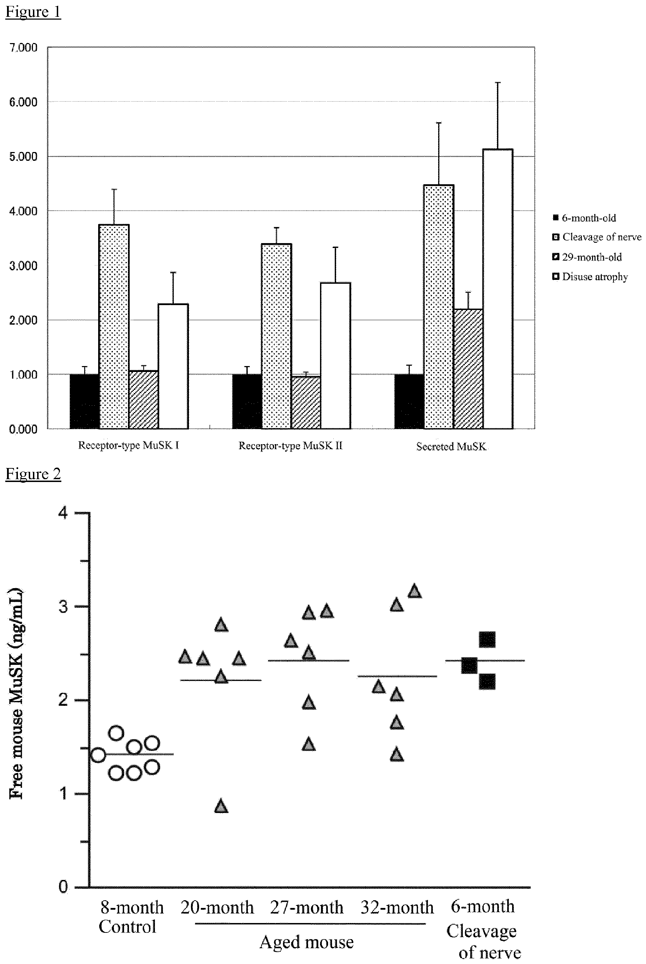

The results are shown in Fig. 1. With respect to the mRNA of the secreted MuSK, the expression increased in the cleavage of the sciatic nerve, the cast immobilization (disuse), and the elderly mice with amyotrophy, in comparison with the normal 6-month-old mice. On the other hand, the mRNA of the receptor-type MuSK did not increase in the elderly mice.

### «Example 4: Measurement of amount of free MuSK protein in mouse serum by AlphaLISA method»

As mice to be measured, 8-month-old mice (control), aged mice (C57BL/6) including 20-month-old mice, 27-month-old mice, and 32-month-old mice, and 6-month-old mice (A/WySnJ) after 11 days from cleavage of the sciatic nerve were prepared, and serum samples (10% serum) were used in the following measurement.

Mice were immunized with a secreted protein consisting of a mouse-derived recombinant MuSK extracellular domain to prepare monoclonal antibodies. Two types of monoclonal antibodies (RM-24 and MH-30) were used to construct an assay system according to an AlphaLISA method (Perkin Elmer). MH-30 (IgG2aκ) was adsorbed by acceptor beads, and RM-24 (IgG2aκ) was labeled with biotin. A standard antigen (recombinant mouse MuSK protein) was diluted with a diluent for standard antigen to prepare a dilution series with 12 steps in a common ratio from 100 ng/mL (the 12th step: a blank). A 96-well plate was used, and 5 µL of the antigen liquid (standard antigen or 5% serum) and 10 µL of 50 µg/mL acceptor beads liquid per well were reacted for 2 hours, and 10 µL of 15 nmol/L biotinylated antibody was added and further reacted for 1 hour. Next, 25 µL of 80 µg/mL streptavidin-adsorbed donor beads were added, and after 30 minutes, the plate was measured using a measurement apparatus (Envision; Perkin Elmer).

The results are shown in Fig. 2. The amounts of the free MuSK protein in sera of the elderly mice and the nerve-cleaved mice increased, in comparison with the normal 8-month-old mice.

### «Example 5: Measurement of amount of free MuSK protein in human serum by AlphaLISA method»

With respect to subjects to be measured, written informed consent was obtained from September, 2012 to October, 2013, with the approval of the ethics committee of the Tokyo Metropolitan Geriatric Hospital and Institute of Gerontology, and cooperating hospitals. Blood was collected from 8 patients (4 males and 4 females, 32-year-old to 78-year-old) with amyotrophic lateral sclerosis (ALS) and 3 patients (2 males and 1 female, 23-year-old to 79-year-old) with anti-acetylcholine receptor (AChR) antibody positive myasthenia gravis (MG).

Mice were immunized with a secreted protein consisting of a human-derived recombinant MuSK extracellular domain to prepare monoclonal antibodies. Two types of monoclonal antibodies (MH-58 and MH-64) were used to construct an assay system according to an AlphaLISA method. MH-64 (IgG2aκ) was adsorbed to acceptor beads, and MH-58 (IgG2aκ) was labeled with biotin. A standard antigen (recombinant mouse MuSK protein) was diluted with a diluent for a standard antigen to prepare a dilution series with 11 steps in a common ratio from 10 ng/mL (the 11th step: a blank). A 96-well plate was used, and 5 µL of the antigen liquid (standard antigen or 100% serum) and 10 µL of 50 µg/mL acceptor beads liquid per well were reacted for 2 hours, and 10 µL of a 15 nmol/L biotinylated antibody was added and further reacted for 1 hour. Next, 25 µL of 80 µg/mL streptavidin-adsorbed donor beads were added, and after 30 minutes, the plate was measured using a measurement apparatus (Envision; Perkin Elmer).

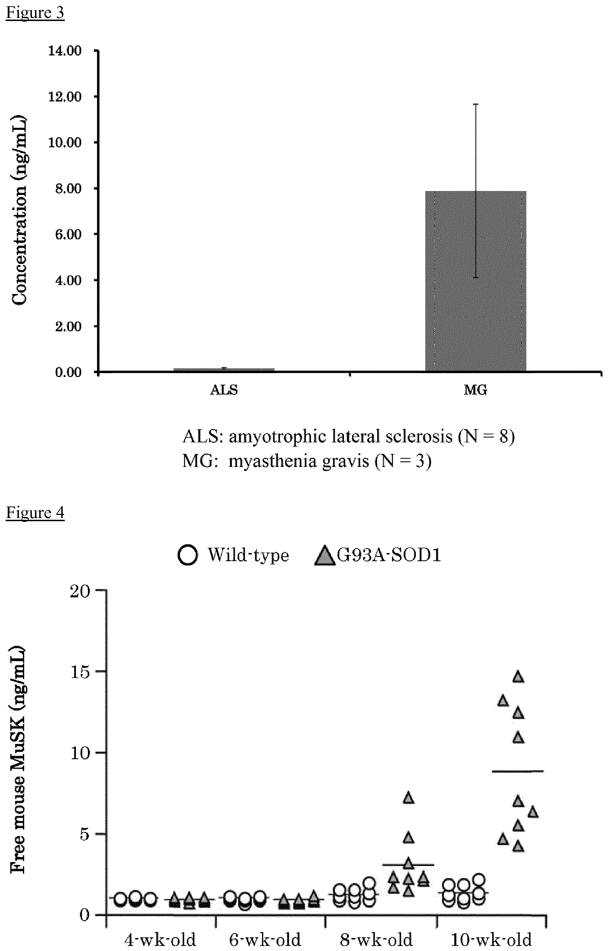

The results are shown in Fig. 3. In the patients with amyotrophic lateral sclerosis (ALS), in which neuroskeletal synapses were lost, and severe amyotrophy was developed, the amount of the free MuSK protein in a serum was remarkably reduced (substantially disappeared).

### «Example 6: Measurement of amount of free MuSK protein in serum of mice with amyotrophic lateral sclerosis (ALS) by AlphaLISA method»

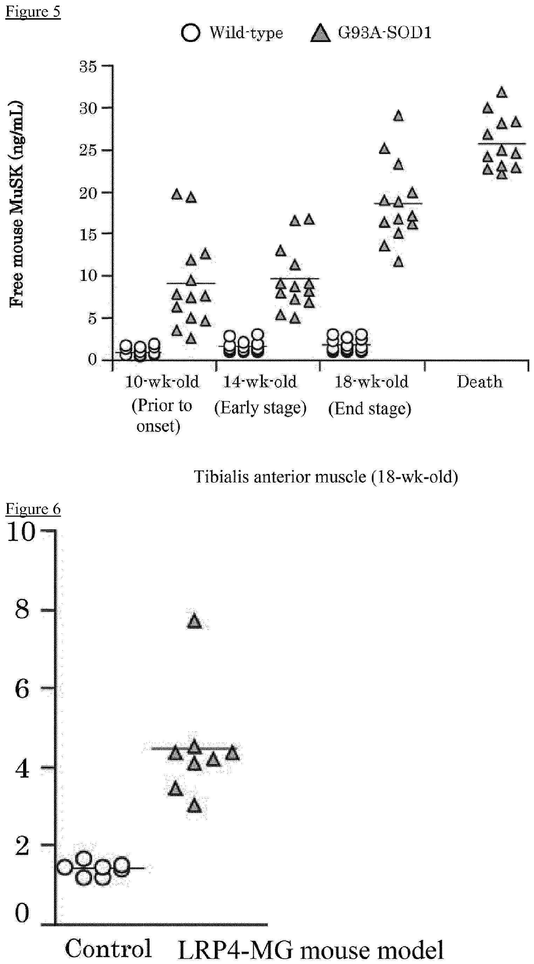

As mice to be measured, wild-type mice (C57BL/6) and ALS mouse models were prepared. Serum samples (5% serum) from 4-week-old mice, 6-week-old mice, 8-week-old mice, and 10-week-old mice (prior to onset), 14-week-old mice (early stage), 18-week-old mice (end stage), and mice at the time of death, were measured in accordance with the procedure described in Example 4. The results are shown in Figs. 4 and 5.

In the wild-type mice, the amount of the free MuSK protein in a serum did not change. However, in the ALS mouse models, the amount of the free MuSK protein in a serum increased prior to the onset, and a further increase was observed in accordance with the progress of the disease. In connection with this, since the course from the onset to death of the mouse model proceeds very early, it dies prior to the end stage of onset in a human (i.e., the state in which severe amyotrophy is developed), and therefore, it is considered that this result is not contradictory to the result of human ALS patients in Example 5.

### «Example 7: Measurement of amount of free MuSK protein in serum of mice with myasthenia gravis (MG) by AlphaLISA method»

As mice to be measured, wild-type mice (C57BL/6) and MG mouse models (EAMG) were prepared. Serum samples (5% serum) of 6-week-old to 8-week-old mice were measured in accordance with the procedure described in Example 4. The results are shown in Fig. 6.

In the MG mouse models, an increase in the amount of the free MuSK protein in a serum was observed in accordance with the onset.

### <<Example 8: Detection of free MuSK protein in serum of mice with amyotrophic lateral sclerosis (ALS)>>

Sera were collected from 18-week-old to 24-week-old ALS mouse models (G93A-SOD1), and 7.7 mL of the serum was subjected to ammonium sulfate fractionation (80% saturation) to obtain 6.5 mL of a supernatant. The supernatant was concentrated to 1.2 mL, using a centrifugal filter unit (Amicon (registered trademark) Ultra, 10 KDa filter; Merck Millipore), and immunoprecipitation was carried out using the mouse monoclonal antibodies prepared in Example 4 (RM-24 and MH-30). The obtained precipitate (hereinafter referred to as immunoprecipitate) was dissolved in 15 µL of an SDS buffer, and the whole amount (15 µL) was analyzed by Western blotting (rabbit anti-mouse MuSK monoclonal antibody No. 2-6).

For comparison, the secreted protein consisting of the mouse-derived recombinant MuSK extracellular domain (recombinant mouse MuSK protein, 100 ng), which was used in Example 4, was simultaneously used in the Western blotting.

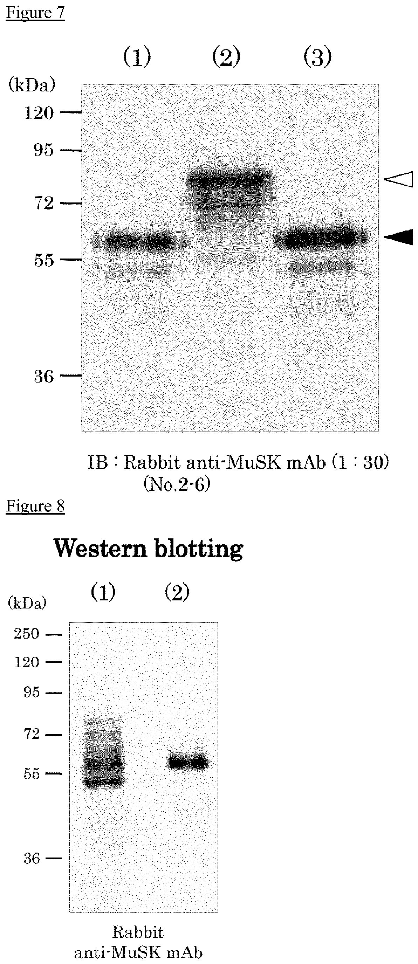

The results are shown in Fig. 7. In Fig. 7, lane 1 shows the immunoprecipitate of the secreted protein consisting of the mouse-derived recombinant MuSK extracellular domain (100 ng), lane 2 shows the immunoprecipitate of the serum from the mice with ALS, and lane 3 shows the immunoprecipitate of the secreted protein consisting of the mouse-derived recombinant MuSK extracellular domain (100 ng).

### «Example 9: Identification of free MuSK protein secreted into culture supernatant of mouse cultured myoblast cell line»

Mouse cultured myoblast cell line C2C12 was seeded in eight 10-cm dishes, and precultured in a DMEM medium containing 10% fetal calf serum (FCS) for 3 days. The medium was replaced with a differentiation medium (a DMEM medium containing 2.5% horse serum (HS)), and each culture supernatant was collected by replacing the medium with a fresh medium at day 1, day 2, day 3, and day 5 after the beginning of the differentiation induction to myotube cells.

Next, 300 mL of the collected culture supernatant was subjected to ammonium sulfate fractionation (80% saturation) to obtain 30 mL of a supernatant. The obtained supernatant was purified using an anti-mouse MuSK antibody column, and finally concentrated using a centrifugal filter unit (Amicon (registered trademark) Ultra, 10 KDa filter; Merck Millipore).

The results of Western blotting (rabbit anti-mouse MuSK monoclonal antibody No.2-6) of the obtained purified fraction are shown in Fig. 8. In Fig. 8, lane 1 shows the purified fraction derived from the culture supernatant of the mouse cultured myoblast cell line, after passing through the antibody column; and lane 2 shows the secreted protein consisting of the mouse-derived recombinant MuSK extracellular domain (for comparison).

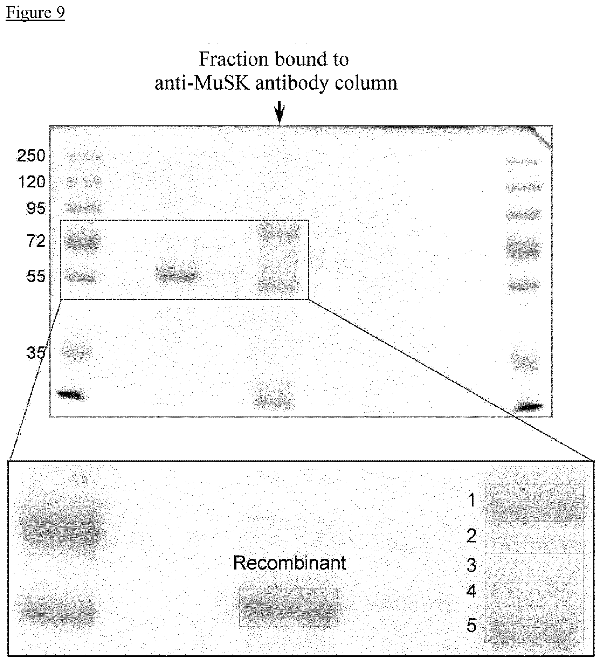

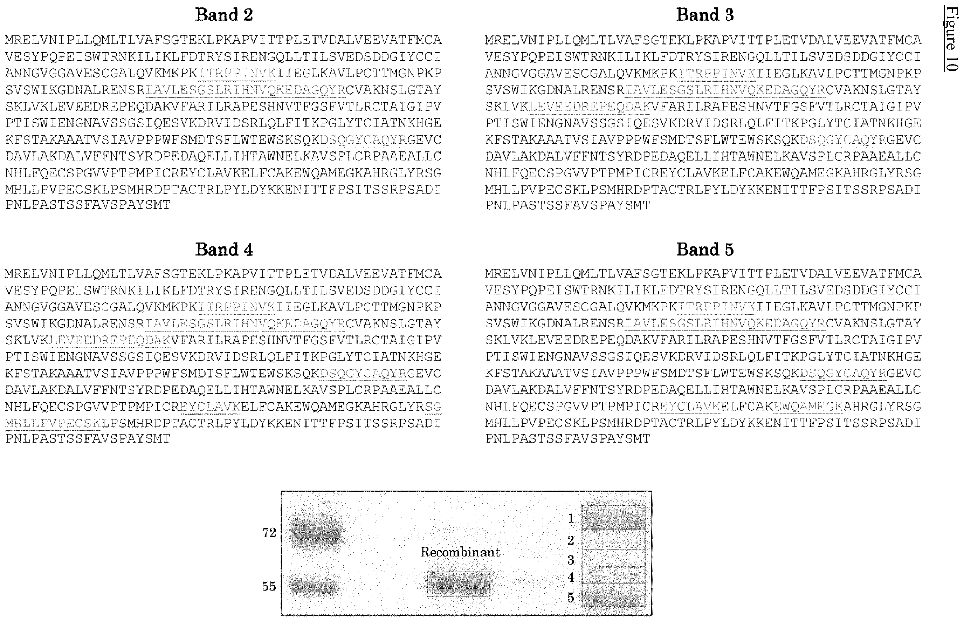

Further, the results of CBB staining of the purified fraction (and the secreted protein consisting of the mouse-derived recombinant MuSK extracellular domain (for comparison)) are shown in Fig. 9. As shown in Fig. 9, the gel corresponding to a region of 85 kDa to 50 kDa after electrophoresis was divided into 5 pieces (Bands 1-5 in Fig. 9) and analyzed by LC-MS/MS. As a result, as shown in Fig. 10, the amino acid sequence (SEQ ID NO: 11) of the extracellular domain of the mouse receptor-type MuSK protein was detected in Bands 2-5, and the purified fraction was confirmed to be a free MuSK protein (a free MuSK protein released into the culture supernatant by cleaving the receptor-type MuSK protein by a protease).

### «Example 10: Reduction of amount of MuSK protein secreted into culture supernatant of mouse cultured myoblast cell line by ADAM inhibition»

Mouse cultured myoblast cell line C2C12 was seeded in dishes, and precultured in a DMEM medium containing 10% FCS for 3 days. The medium was replaced with a differentiation medium (a DMEM medium containing 2.5% HS) with predetermined amounts of an ADAM (a disintegrin and metalloproteinase) inhibitor GM6001 (Calbiochem, #364206), and further cultured for 22 hours. After the cultivation, the amount of the free MuSK protein contained in each culture supernatant was measured.

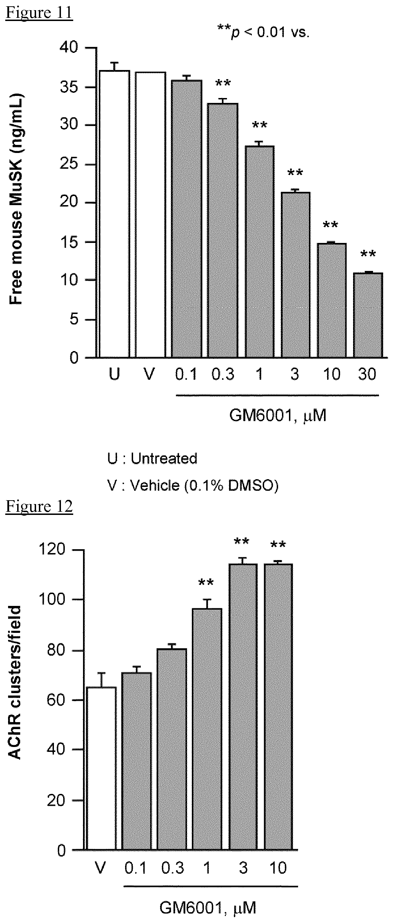

The results are shown in Fig. 11. It was confirmed that the amount of the free MuSK protein contained in the culture supernatant was reduced by the addition of GM6001. This result indicates that at least part of the free MuSK protein in the culture supernatant is derived from the receptor-type MuSK protein released into the culture supernatant by cleaving it by a protease.

### «Example 11: Increase of acetylcholine receptor (AChR) clustering in mouse cultured muscle cell line by ADAM inhibitor»

GM6001, which was dissolved in DMSO, was diluted with a differentiation medium (a DMEM medium containing 2.5% HS), and 0.4 ml/well of the GM6001 solution was added to mouse C2C12 myotube cells, which had been differentiated for 3 days in 24-well plates. After 22 hours of cultivation, culture supernatants were collected, and AChR clustering staining was carried out by culturing the cells in a differentiation medium containing 40 nM Alexa 488-labeled α-bungarotoxin for 1 hour.

The results are shown in Fig. 12. AChR clustering is an indicator of synaptogenesis, and it was shown that synaptogenesis was promoted by an ADAM inhibitor.

### INDUSTRIAL APPLICABILITY

The biomarker of the present invention can be used in the diagnosis of aging or amyotrophy.

### FREE TEXT IN SEQUENCE LISTING

The nucleotide sequences of SEQ ID NOS: 5 to 10 in the sequence listing are primer sequences.

## Claims

1. An in vitro method for determining a state of aging or amyotrophy, said method comprising the step of measuring a MuSK protein which is present in a free state, or a mRNA for a non-receptor-type MuSK protein, in a sample obtained from a subject, wherein the MuSK protein which is present in a free state is a non-receptor-type MuSK protein and/or a truncated MuSK protein.

2. The method according to claim 1, wherein the determination of a state of aging or amyotrophy is a determination of a degree of aging, an early diagnosis of amyotrophy, or a detection of severe amyotrophy.

3. A biomarker for determining a state of aging or amyotrophy, said biomarker comprising a MuSK protein which is present in a free state, or a mRNA for a non-receptor-type MuSK protein, wherein the MuSK protein which is present in a free state is a non-receptor-type MuSK protein and/or a truncated MuSK protein.

4. The biomarker according to claim 3, wherein the determination of a state of aging or amyotrophy is a determination of a degree of aging, an early diagnosis of amyotrophy, or a detection of severe amyotrophy.

5. An in vitro method for determining the efficacy of therapeutic or preventive effects on aging or amyotrophy, comprising the step of measuring a MuSK protein which is present in a free state, or a mRNA for a non-receptor-type MuSK protein, in a sample obtained from a subject, wherein the MuSK protein which is present in a free state is a non-receptor-type MuSK protein and/or a truncated MuSK protein.

6. A method of screening for a therapeutic or preventive agent for aging or amyotrophy, said method comprising the steps of:
- administering a test substance to an non-human animal model of aging or amyotrophy, or a cell line of the non-human animal model;
- measuring a MuSK protein which is present in a free state, or an mRNA for a non-receptor-type MuSK protein, in a sample obtained from the non-human animal model, or a culture supernatant of the cell line, wherein the MuSK protein which is present in a free state is a non-receptor-type MuSK protein and/or a truncated MuSK protein; and
- selecting, as a candidate for a therapeutic or preventive agent for aging or amyotrophy, a test substance in which the amount of the MuSK protein which is present in a free state, or the expression level of the mRNA for the non-receptor-type MuSK is reduced.

7. A protein selected from the following (a) and (b):
(a) a protein comprising the amino acid sequence of SEQ ID NO: 2 or SEQ ID NO: 12; and
(b) a protein consisting of an amino acid sequence that has at least 80% identity to the amino acid sequence of SEQ ID NO: 2 or SEQ ID NO: 12, and having secreted MuSK protein activity.

8. A polynucleotide encoding the protein of claim 7.

9. A vector comprising the polynucleotide of claim 8.

10. A cell transformed with the vector of claim 9.

11. A kit of screening for a therapeutic or preventive agent for aging or amyotrophy, said kit comprising the cell of claim 10.

## Patentansprüche

1. Ein *in-vitro-*Verfahren zur Bestimmung des Stadiums der Alterung oder des Muskelschwunds, wobei dieses Verfahren die Stufe der Messung eines im freien Zustand vorliegenden MuSK-Proteins oder einer mRNA für ein Nicht-Rezeptor-MuSK-Protein in einer von einem Lebewesen erhaltenen Probe umfasst, wobei das im freien Zustand vorliegende MuSK-Protein ein Nicht-Rezeptor-MuSK-Protein und/oder ein trunkiertes MuSK-Protein ist.

2. Das Verfahren nach Anspruch 1, wobei die Bestimmung des Stadiums der Alterung oder des Muskelschwunds eine Bestimmung des Grades der Alterung, eine frühzeitige Diagnose des Muskelschwunds oder der Nachweis eines ernsthaften Muskelschwunds ist.

3. Ein Biomarker zur Bestimmung des Stadiums der Alterung oder des Muskelschwunds, wobei dieser Biomarker ein im freien Zustand vorliegendes MuSK-Protein oder eine mRNA für ein Nicht-Rezeptor-MuSK-Protein umfasst, wobei das im freien Zustand vorliegende MuSK-Protein ein Nicht-Rezeptor-MuSK-Protein und/oder ein trunkiertes MuSK-Protein ist.

4. Der Biomarker nach Anspruch 3, wobei die Bestimmung des Stadiums der Alterung oder des Muskelschwunds eine Bestimmung des Grades der Alterung, eine frühzeitige Diagnose des Muskelschwunds oder der Nachweis eines ernsthaften Muskelschwunds ist.

5. Ein *in-vitro-*Verfahren zur Bestimmung der Wirksamkeit therapeutischer oder präventiver Maßnahmen bei Alterung oder Muskelschwund, welches die Stufe der Messung eines im freien Zustand vorliegenden MuSK-Proteins oder einer mRNA für ein Nicht-Rezeptor-MuSK-Protein in einer von einem Lebewesen erhaltenen Probe umfasst, wobei das im freien Zustand vorliegende MuSK-Protein ein Nicht-Rezeptor-MuSK-Protein und/oder ein trunkiertes MuSK-Protein ist.

6. Ein Verfahren zum Screening nach einem therapeutischen oder präventiven Mittel gegen das Altern oder den Muskelschwund, welches die Stufen:
- Verabreichen einer Testsubstanz an ein nichthumanes Tiermodell für die Alterung oder den Muskelschwund oder an eine Zelllinie von dem nicht-humanen Tiermodell,
- Messen eines im freien Zustand vorliegenden MuSK-Proteins oder einer mRNA für ein Nicht-Rezeptor-MuSK-Protein in einer von dem nicht-humanen Tiermodell oder einem Kulturüberstand der Zelllinie erhaltenen Probe, wobei das im freien Zustand vorliegende MuSK-Protein ein Nicht-Rezeptor-MuSK-Protein und/oder ein trunkiertes MuSK-Protein ist, und
- Auswählen einer Testsubstanz, bei welcher der Spiegel des im freien Zustand vorliegenden MuSK-Proteins oder das Expressionsniveau der mRNA für das Nicht-Rezeptor-MuSK-Protein verringert ist, als Kandidat für ein therapeutisches oder präventives Mittel gegen Alterung oder Muskelschwund umfasst.

7. Ein Protein, das aus den folgenden a) und b):
a) ein Protein, das die Aminosäuresequenz mit der SEQ ID NO: 2 oder SEQ ID NO: 12 umfasst, und
b) ein Protein, das aus einer Aminosäuresequenz besteht, die eine mindestens 80%ige Identität mit der Aminosäuresequenz mit der SEQ ID NO: 2 oder SEQ ID NO: 12 und eine Sekretierte-MuSK-Protein-Aktivität besitzt,
ausgewählt ist.

8. Ein das Protein nach Anspruch 7 codierendes Polynucleotid.

9. Ein das Polynucleotid nach Anspruch 8 umfassender Vektor.

10. Eine mit dem Vektor nach Anspruch 9 transformierte Zelle.

11. Ein Kit für das Screening nach einem therapeutischen oder präventiven Mittel gegen das Altern oder den Muskelschwund, wobei dieser Kit die Zelle nach Anspruch 10 umfasst.

## Revendications

1. Procédé in vitro pour déterminer un état de vieillissement ou d'amyotrophie, ledit procédé comprenant l'étape consistant à mesurer une protéine MuSK qui est présente à l'état libre, ou bien à mesurer un acide ribonucléique messager - ARNm - pour une protéine MuSK du type non-récepteur, dans un échantillon prélevé sur un sujet, échantillon dans lequel la protéine MuSK, qui est présente à l'état libre, est une protéine MuSK du type non-récepteur et/ou une protéine MuSK tronquée.

2. Procédé selon la revendication 1, dans lequel la détermination d'un état de vieillissement ou d'amyotrophie est une détermination d'un degré de vieillissement, un diagnostic précoce d'amyotrophie, ou bien une détection d'une amyotrophie sévère.

3. Biomarqueur pour déterminer un état de vieillissement ou d'amyotrophie, ledit biomarqueur comprenant une protéine MuSK qui est présente à l'état libre, ou bien comprenant un ARNm pour une protéine MuSK du type non-récepteur, biomarqueur dans lequel la protéine MuSK, qui est présente à l'état libre, est une protéine MuSK du type non-récepteur et/ou une protéine MuSK tronquée.

4. Biomarqueur selon la revendication 3, dans lequel la détermination d'un état de vieillissement ou d'amyotrophie est une détermination précoce d'une détection d'une amyotrophie sévère.

5. Procédé in vitro pour déterminer l'efficacité d'effets thérapeutiques ou préventifs sur le vieillissement ou sur l'amyotrophie, ledit procédé comprenant l'étape consistant à mesurer une protéine MuSK qui est présente à l'état libre, ou bien à mesurer un ARNm pour une protéine MuSK du type non-récepteur, dans un échantillon prélevé sur un sujet, échantillon dans lequel la protéine MuSK, qui est présente à l'état libre, est une protéine MuSK du type non-récepteur et/ou une protéine MuSK tronquée.

6. Procédé de dépistage pour un agent thérapeutique ou préventif pour le vieillissement ou pour l'amyotrophie, ledit procédé comprenant les étapes consistant :
- à administrer une substance test à un modèle animal non humain de vieillissement ou d'amyotrophie, ou bien à administrer une lignée cellulaire du modèle animal non humain ;
- à mesurer une protéine MuSK qui est présente à l'état libre, ou bien à mesurer un ARNm pour une protéine MuSK du type non-récepteur, dans un échantillon prélevé sur le modèle animal non humain, ou bien à mesurer un surnageant de cultures de la lignée cellulaire, où la protéine MuSK, qui est présente à l'état libre, est une protéine MuSK du type non-récepteur et/ou une protéine MuSK tronquée ; et
- à sélectionner, comme candidat pour un agent thérapeutique ou préventif pour le vieillissement ou pour l'amyotrophie, une substance test dans laquelle est réduite la quantité de protéine MuSK qui est présente à l'état libre, ou bien substance test dans laquelle le niveau d'expression de l'ARNm pour la protéine MuSK du type non-récepteur est réduit.

7. Protéine sélectionnée parmi les cas suivants (a) et (b):
(a) une protéine comprenant la séquence d'acides aminés de SEQ ID NO : 2 ou de SEQ ID NO : 12 ; et
(b) une protéine se composant d'une séquence d'acides aminés, séquence dont l'identité est égale à au moins 80 % par rapport à celle de la séquence d'acides aminés de SEQ ID NO : 2 ou de SEQ ID NO : 12, et ayant sécrété une activité de protéine MuSK.

8. Polynucléotide codant pour la protéine de la revendication 7.

9. Vecteur comprenant le polynucléotide de la revendication 8.

10. Cellule transformée avec le vecteur de la revendication 9.

11. Kit de dépistage pour un agent thérapeutique ou préventif pour le vieillissement ou l'amyotrophie, ledit kit comprenant la cellule de la revendication 10.
